(19) Europäisches Patentamt
European Patent Office
Office européen des brevets

(11) Publication number: **0 367 895 A1**

(12) # EUROPEAN PATENT APPLICATION

(21) Application number: 89103931.5

(22) Date of filing: 06.03.89

(51) Int. Cl.[5]: C07D 405/06, C07D 239/42, C07D 239/26, C07D 401/04, A61K 31/505

Claims for the following Contracting States: ES + GR

(30) Priority: 06.10.88 US 254514

(43) Date of publication of application: 16.05.90 Bulletin 90/20

(84) Designated Contracting States: AT BE CH DE ES FR GB IT LI LU NL SE

(71) Applicant: SANDOZ AG
Lichtstrasse 35
CH-4002 Basel(CH)
(84) BE CH ES FR GB IT LI LU NL SE

Applicant: SANDOZ-PATENT-GMBH
Humboldtstrasse 3
D-7850 Lörrach(DE)
(84) DE

Applicant: SANDOZ-ERFINDUNGEN
Verwaltungsgesellschaft m.b.H.
Brunner Strasse 59
A-1235 Wien(AT)
(84) AT

(72) Inventor: Kathawala, Faizulla Gulamhusein
39 Woodland Avenue
Mountain Lakes, NJ 07946(US)

(54) **Pyrimidinyl-substituted hydroxyacids, lactones and esters and pharmaceutical compositions containing them.**

(57) Compounds of formula I

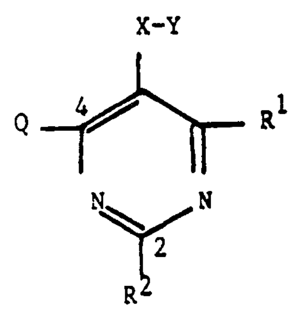

I

wherein $R^1$, $R^2$, Q, X and Y have various significances,
in free acid form, or in the form of an ester or δ-lactone thereof, or in salt form as appropriate, are described.
They are indicated for use as hypolipoproteinemic and anti-atherosclerotic agents. They may be obtained by reduction, hydrolysis, deprotection or oxidation of appropriate starting compounds, and variously interconverted by e.g. hydrolysis, salt formation, esterification and/or lactonisation.

EP 0 367 895 A1

# PYRIMIDINYL-SUBSTITUTED HYDROXYACIDS, LACTONES AND ESTERS AND PHARMACEUTICAL COMPOSITIONS CONTAINING THEM

This invention relates to pyrimidinyl-substituted hydroxy-acids, lactones and esters and pharmaceutical compositions containing them. These compounds are cholesterol biosynthesis inhibitors, and as such are indicated for use in the treatment of hyperlipoproteinemia, and hence in the treatment of hyperlipoproteinemia, and hence in the treatment of atherosclerosis.

The invention concerns the compounds of formula I,

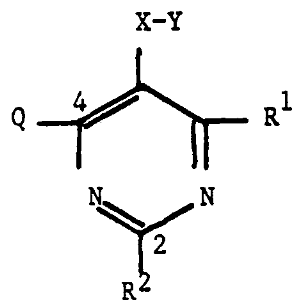

I

wherein
either
$R^1$ and $R^2$ independently are:
$C_{1-6}$alkyl not containing an asymmetric carbon atom;
$C_{3-6}$cycloalkyl; or

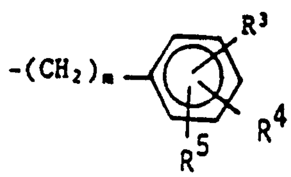

wherein
m is 0, 1, 2 or 3;
$R^3$ is hydrogen, $C_{1-3}$alkyl, n-butyl, i-butyl, t-butyl, $C_{1-3}$alkoxy, n-butoxy, i-butoxy, trifluoromethyl, fluoro, chloro, phenoxy or benzyloxy;
$R^4$ is hydrogen, $C_{1-3}$alkyl, $C_{1-3}$alkoxy, trifluoromethyl, fluoro, chloro, phenoxy or benzyloxy; and
$R^5$ is hydrogen, $C_{1-2}$alkyl, $C_{1-2}$alkoxy, fluoro or chloro;
with the provisos that
- not more than one of $R^3$ and $R^4$ is trifluoromethyl;
- not more than one of $R^3$ and $R^4$ is phenoxy; and
- not more than one of $R^3$ and $R^4$ is benzyloxy;
or
$R^1$ is as defined above and
$R^2$ is: benzyloxy
benzylthio;
$-N(R^8)_2$ wherein either each $R^8$ independently is $C_{1-4}$alkyl not containing an asymmetric carbon atom or both $R^8$ together with the nitrogen atom form part of a 5-,6- or 7-membered optionally substituted ring optionally containing one or more further heteroatoms (ring B); or
Q wherein Q is as defined below;
Q is Q′ or Q″ wherein
Q′ is a heterocyclic group optionally mono- or independently disubstituted by $C_{1-2}$alkyl or $C_{1-2}$alkoxy and
Q″ is either $Q''_a$ wherein $Q''_a$ is

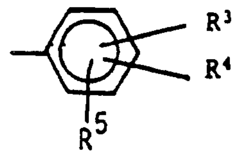

wherein
$R^3$, $R^4$ and $R^5$ are as defined above, including the provisos thereto,
or $Q''_b$ wherein $Q''_b$ is

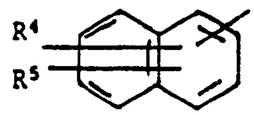

wherein
$R^4$ and $R^5$ are as defined above;
X is either ethylene or vinylene; and
Y is: - a group $Y'$ of formula

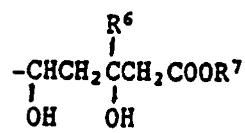

wherein
$R^6$ is hydrogen or $C_{1-3}$alkyl; and
$R^7$ is hydrogen, an ester group ($R^{7'}$) or a cation (M);
- a group $Y''$ of formula

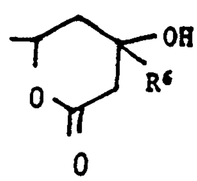

wherein $R^6$ is as defined above; or
- group $Y'''$ of formula

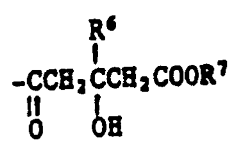

wherein
$R^6$ and $R^7$ are as defined above;
with the proviso that when Y is a group $Y'''$,
then X is vinylene and/or
$R^6$ is $C_{1-3}$alkyl,
in free acid form, or in the form of an ester or δ-lactone thereof, or in salt form as appropriate.

The term "not containing an asymmetric carbon atom" includes, but is not limited to methyl, ethyl, n-propyl, i-propyl, n-butyl, t-butyl, l-ethylpropyl, neopentyl and n-hexyl.

A first subgroup of compounds of the invention comprises the compounds of formula Ia ($Y = Y'$):

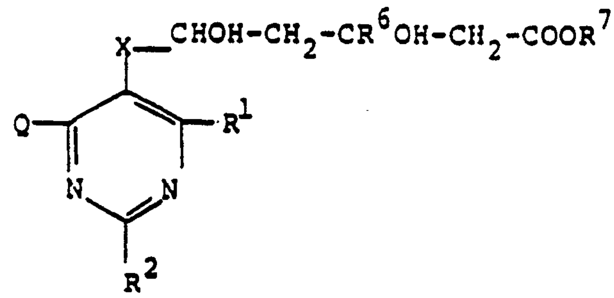

Ia

A second subgroup of compounds comprises the compounds of formula Ib (Y = Y″):

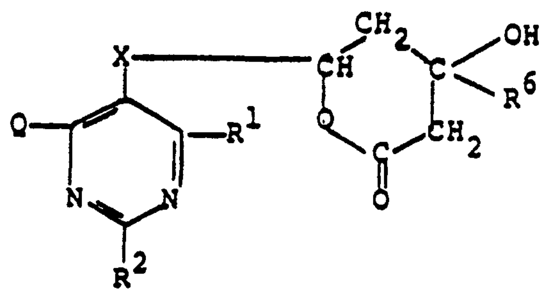

Ib

A third subgroup of compounds comprises the compounds of formula Ic (Y = Y‴):

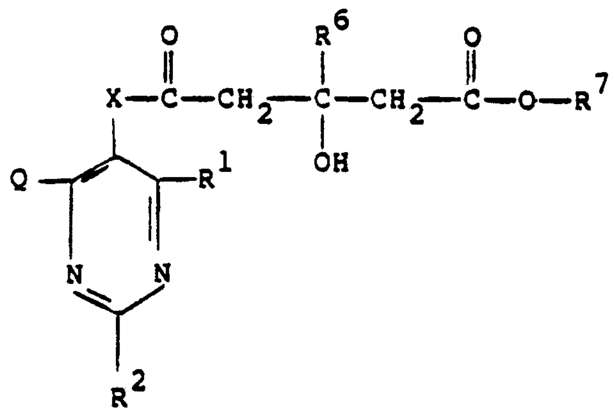

Ic

In formulae Ia, Ib and Ic $R^1$, $R^2$, $R^6$, $R^7$, X and Q are as defined above.
A further subgroup is the compounds of formula I′ (Q = Q″a):

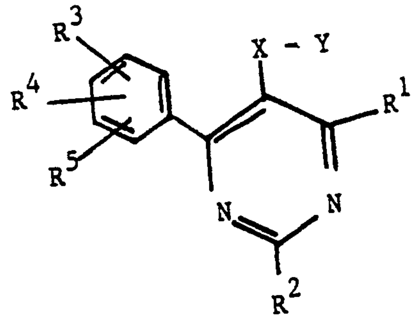

I′

A further subgroup is the compounds of formula I″ (Q = Q′):

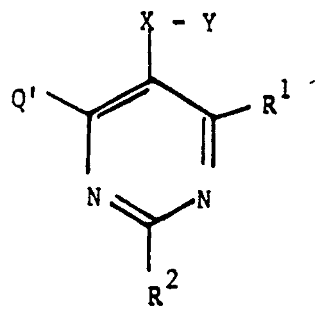

I"

In formulae $I'$ and $I''$ $R^1$ to $R^5$, $Q'$, X and Y are as defined above.

Further subgroups are compounds of formula I

a) in which $R^2$ is unsubstituted phenyl;

b) in which $R^1$, $R^2$ and especially Q is p-fluorophenyl;

c) in which $R^1$ is isopropyl, especially those wherein both $R^1$ and $R^2$ are isopropyl; and

d) in which $R^2$ is dimethylamino or morpholinyl.

Preferably no more than one of $R^1$ or $R^2$ is a group containing unsubstituted or substituted phenyl or $Q'$; in particular, $R^1$ preferably is other than a group containing unsubstituted or substituted phenyl or $Q'$.

A first subgroup of the compounds of formula Ia is the compounds of formula $Ia^1$ ($R^7$ = $R^{7'}$):

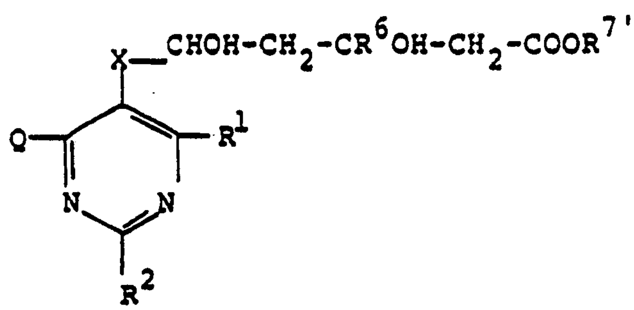

$Ia^1$

A second subgroup of compounds of formula Ia is the compounds of formula $Ia^2$ ($R^7$ = M):

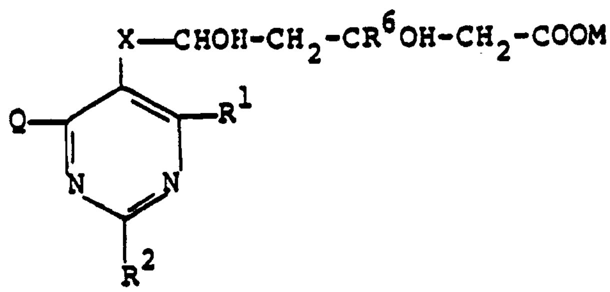

$Ia^2$

A third subgroup of compound of formula Ia is the compounds of formula $Ia^3$ ($R^7$ = H):

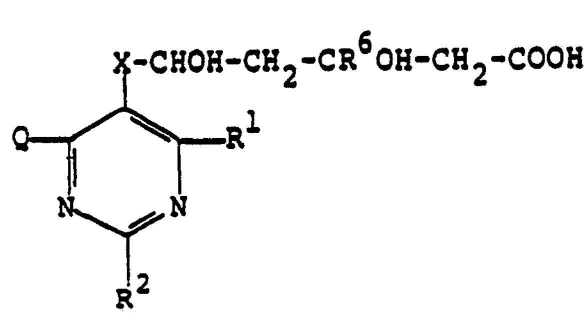

$Ia^3$

In subgroup $Ia^1$, $Ia^2$ and $Ia^3$ $R^1$, $R^2$, $R^6$, $R^{7'}$, X, M and Q are as defined above.

Further subgroups of compounds of formula I are the compounds of formula Ia and Ib in which Q is $Q''a$, those in which neither $R^1$ nor $R^3$ is tertiary $C_{4-6}$alkyl, those in which $R_2$ is other than $Q'$ and $Q''a$ and those in which at least one of $R^1$ and $R^2$ is other than phenyl and substituted phenyl and (1) $R^3$ and $R^4$ both are hydrogen and $R^5$ is fluoro; (2) $R^1$ is isopropyl; and (3) $R^2$ is unsubstituted phenyl.

Suitable ester forms include physiologically acceptable esters, e.g. physiologically acceptable and hydrolyzable esters. By the term "physiologically acceptable and hydrolyzable esters" is meant compounds having a group which, together with the carboxyl radical to which it is attached, forms an ester group which is acceptable and hydrolyzable under physiological conditions to yield a compound of formula I wherein $R^{7'}$ is hydrogen and an alcohol which itself is physiologically acceptable, i.e., non-toxic at the desired dosage level. Examples of such groups are $C_{1-3}$alkyl, n-butyl, i-butyl, t-butyl and benzyl, collectively referred to as $R^{7''}$.

Suitable salt forms include pharmaceutically acceptable salts, i.e. salts with cations which are pharmaceutically acceptable, e.g. alkali cations such as sodium, potassium or ammonium.

$R^1$ preferably is alkyl as defined above.

$R^2$ preferably is alkyl as defined above, a phenyl-containing group as defined above, or a group $-N(R^8)_2$ as defined above, especially a group $-N(R^8)_2$.

Q preferably is $Q'$ or $Q''_a$ as defined above, especially $Q''_a$.

X preferably is vinylene.

Y preferably is a group $Y'$ or $Y''$ as defined above, especially a group $Y'$.

$C_{1-6}$alkyl preferably is of 1 to 4, especially of 3 carbon atoms.

$C_{3-6}$cycloalkyl preferably is of 3, 5 or 6, especially of 5 carbon atoms.

m preferably is 0 or 1, especially 1.

$R^3$, $R^4$ and/or $R^5$ preferably are hydrogen, fluorine or methyl.

$R^8$ preferably is $C_{1-2}$alkyl, especially methyl, or part of a heterocycle containing a further oxygen heteroatom.

$N(R^8)_2$ preferably is di($C_{1-2}$)alkylamino or 4-morpholinyl.

$Q'$ preferably is pyridyl, especially 4-pyridyl.

$Q''$ preferably is $Q''_a$.

Vinylene is especially (E)-CH=CH-.

$R^6$ preferably is hydrogen.

$R^7$ preferably is $R^{7'}$ or M.

$R^{7'}$ preferably is methyl or ethyl.

M preferably is sodium, potassium or ammonium, especially sodium.

A further group of compounds of the invention is the compounds of formula $I_s$.

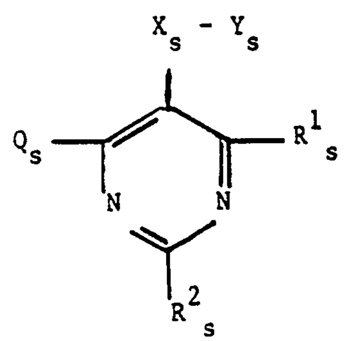

$I_s$

wherein

$R^1_s$ is ($C_{1-3}$)alkyl,

$R^2_s$ is ($C_{1-3}$)alkyl; phenyl; 4-morpholinyl; or di($C_{1-2}$)alkylamino;

$Q_s$ is phenyl optionally monosubstituted in the 4-position by fluorine or disubstituted in the 3-position and in the 4-or 5-position by $C_{1-2}$alkyl; or 4-pyridyl;

$X_s$ is (E)-vinylene; and

$Y_s$ is - a group $Y'_s$ of formula $-CH(OH)CH_2CH(OH)CH_2COOR^7_s$

wherein $R^7_s$ is hydrogen, ($C_{1-4}$)alkyl or

an alkali cation; or

- a group $Y''_s$ of formula

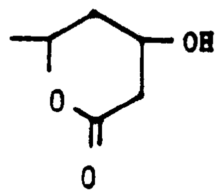

It is to be appreciated that formula I covers compounds of the invention in free acid form (compounds of formula Ia or Ic wherein $R^7$ is H), in salt form (compounds of formula Ia or Ic wherein $R^7$ is a cation) or in ester form (compounds of formula Ia or Ic wherein $R^7$ is an ester group, or compounds of formula Ib), as appropriate, whereby the compounds of formula Ib are to be viewed as being a particular case of compounds of formula Ia in ester form, namely, in cyclic ester (δ-lactone) form.

Salts may be prepared in conventional manner from free acids, lactones and esters and vice-versa. Salts with alkali caticns, i.e. sodium, potassium and ammonium, particularly sodium salts are preferred.

It will thus be appreciated that compounds $Ia^1$, $Ia^2$, $Ia^3$, Ib and Ic can be interconverted by conventional means in the art. For example a compound $Ia^3$ can be lactonised to form the corresponding compound Ib. Likewise a compound Ib can be saponified to its corresponding compound $Ia^2$ which can in turn be esterified to a compound $Ia^1$ or neutralized e.g. by treatment with an acid to form its corresponding compound $Ia^3$. Compounds Ic are obtainable by oxidizing the hydroxy group of a corresponding 5-hydroxy compound of formula I by means conventionally employed in oxidizing e.g. an allylic alcohol to a carbonyl, e.g. using $MnO_2$ or 2,3-dichloro-5,6-di-cyano-1,4-benzoquinone (DDQ). Analogous reactions are reported in the literature.

The above-described interconversions are conveniently represented in Reaction Scheme A, below, wherein Q, X, $R^1$, $R^2$, M, $R^6$ and $R^{7'}$ are as defined above.

For example, lactonisation to a compound of formula Ib is accomplished by heating a compound of formula Ia (in which $R^7 = H$) in an inert organic solvent, e.g. an aromatic hydrocarbon, such as toluene, at from about 80° to about 140°C, for example at the reflux temperature of the reaction medium; or by treating with a lactonisation agent, e.g. a water-soluble carbodiimide such as N-cyclohexyl-N′-[2′-(methylmorpholinium)ethyl]carbodiimide p-toluenesulfonate, i.e. 1-cyclohexyl-3-(2-morpholino)ethylcarbo diimide metho-p-toluenesulfonate in an inert organic solvent, e.g. methylene chloride, at moderate temperatures, e.g. 20 to 40°C.

Saponification of a compound of formula Ib, Ic wherein $R^7$ is $R^{7'}$, or $Ia^1$ to obtain a corresponding compound of formula $Ia^2$, or Ic wherein $R^7$ is M, is achieved by treatment with aqueous alkali metal base, e.g. sodium hydroxide, preferably in a water-miscible co-solvent, e.g. ethanol or dioxane, at moderate temperatures, e.g. from about 0° to 100°C, e.g. at about 75°C. Where a product is desired in which $R^7$ is hydrogen, i.e. the free acid form (a compound of formula $Ia^3$, or Ic wherein $R^7$ is H), such is obtained by acidifying the salt form (where $R^7 = M$) by conventional means, e.g. by addition of dilute hydrochloric acid.

Esterification is also conventional, e.g. employing a large excess of a compound $R^{7'}$-OH wherein $R^{7'}$ is as defined above, at 20°C to 40°C, optionally in a solvent (especially when $R^{7'}$-OH is not liquid) and in the presence of a catalytic amount of an acid such as p-toluenesulfonic acid. Where methyl esters are required these can also be obtained e.g. using diazomethane in an anhydrous inert ether. solvent such as tetrahydrofuran.

Reaction Scheme A

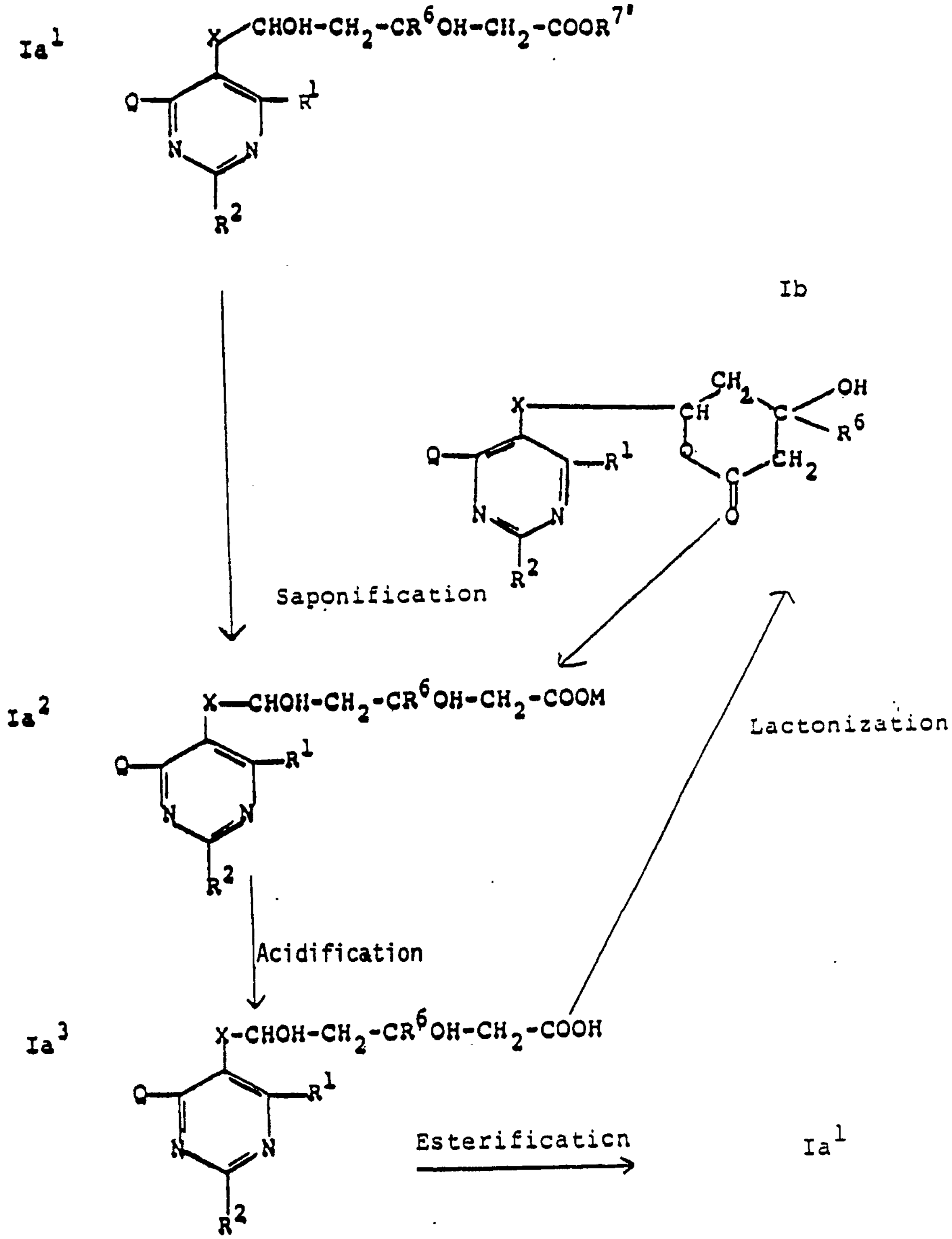


Preferred compounds of formula I are compounds of formula I in stereochemically homogenous form, in particular those in which X is (E)-CH=CH-, i.e. trans olefins, and Y is Y′ having high optical purity, preferably in the erythro (3R, 5S) configuration. Such products may be obtained by saponification and acidification (as described in reaction scheme A) of a corresponding high isomeric purity compound of

formula Ib (see process variant c).

One especially preferred embodiment of the invention is the compounds of formula:

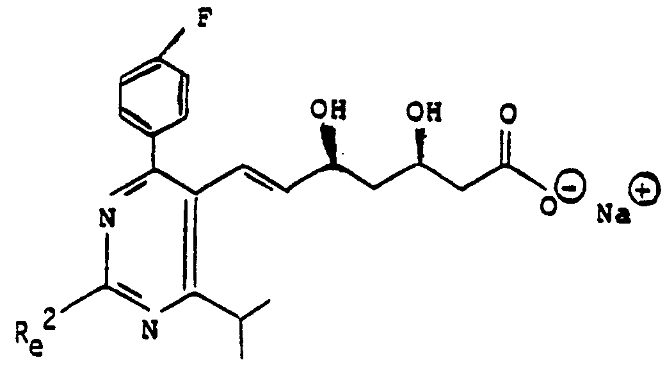

wherein $R_e^2$ is dimethylamino or 4-morpholinyl.

As is self-evident to those in the art, each compound of formula Ia and Ib has two centers of asymmetry (the two carbon atoms bearing the hydroxy groups in the group of formula $Y'$ and the carbon atom bearing the hydroxy group and the carbon atom having the free valence in the group of formula $Y''$ and, therefore, there are four stereoisomeric forms (enantiomers) of each compound (two racemates or pairs of diastereoisomers), provided that M or $R^{7'}$ do not themselves contain any further center of asymmetry. The four stereoisomers may be designated as the R,R; R,S; S,R and S,S enantiomers. Each compound of formula Ic has one center of asymmetry and therefore, there are two stereoisomeric forms (enantiomers) of each compound, provided the M or $R^{7'}$ do not themselves contain any further center of asymmetry. In addition, when X is olefinic, i.e. an ethylenically unsaturated position, then the two hydrogen atoms thereof can be in cis (Z) or trans (E) relationship to each other.

When X is olefinic, it is preferred that the moiety Y be in trans relationship to the pyrimidinyl nucleus. It is also preferred that when Y is a lactone ring, then the 6-hydrogen atom be in trans relationship to the $R^6$ group. Such trans lactone moiety can be formed when the 3- and 5-hydroxy functions on the 3,5-diol-ester moiety of formula Ia are in erythro relationship.

As is evident to those in the art, the configuration remains unchanged, a racemic threo 3,5-dihydroxycarboxylic acid yields a racemic cis lactone (two stereoisomers) and a racemic erythro 3,5-dihydroxycarboxylic acid yields a racemic trans lactone (two stereoisomers). Use of a mixture of threo and erythro 3,5-dicarboxylic acid yields a mixture of cis and trans lactones (all four possible diastereoisomers). Likewise if a single enantiomer of the 3,5-dihydroxycarboxylic acid is utilized, a single enantiomer of the lactone is obtained. For example, lactonisation of a 3R,5S erythro dihydroxycarboxylic acid yields the 4R,6S isomer.

The products described herein may be recovered and purified, where such is desired by conventional means, such as by crystallization, distillation or chromatographic techniques such as column or thin layer chromatography (TLC), e.g. silica gel column chromatography. Where appropriate, intermediates can be employed directly in a subsequent reaction.

Mixtures of stereoisomers (cis, trans and optical) may be separated by conventional means at whatever stage of synthesis is appropriate. Such methods include recrystallization, chromatography, formation of esters with optically pure acids and alcohols or of amides and salts (cf. also Sommer et al. J.A.C.S. 80, 3271 [1985]) with subsequent reconversion under retention of optical purity.

The compounds of formula I in free acid form, or in the form of an ester or δ-lactone thereof, or in salt form as appropriate, may be prepared by a process comprising

a) when $R^6$ is hydrogen

reducing a corresponding compound of formula AAA

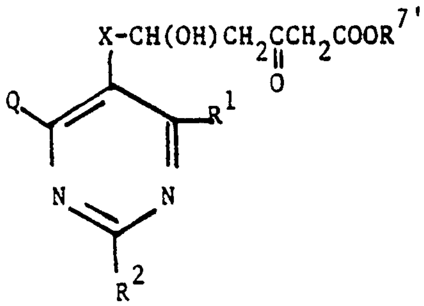

AAA

wherein $R^1$, $R^2$, Q, X and $R^{7'}$ are as defined above, or

b) when $R^6$ is $(C_{1-3})$alkyl,

hydrolyzing a corresponding compound of formula BBB

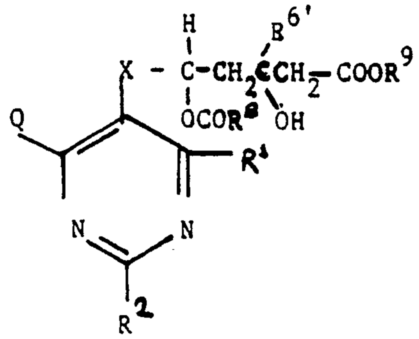

BBB

wherein $R^1$, $R^2$, Q and X are as defined above, $R^{6'}$ is $(C_{1-3})$alkyl and $R^8$ and $R^9$ are part of an ester-forming group, or

c) when $R^6$ is hydrogen and X is (E)-CH=CH-,

deprotecting a corresponding compound of formula CCC

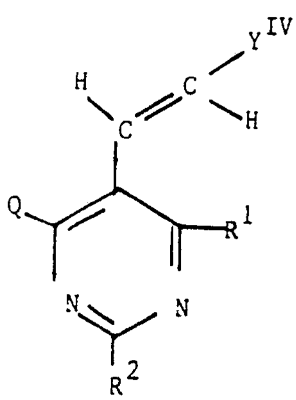

CCC

wherein $R^1$, $R^2$ and Q are as defined above and $Y^{IV}$ is

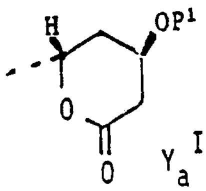

$Y_a^{IV}$

or

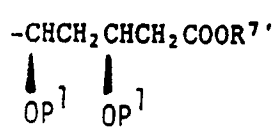

$Y_b^{IV}$

wherein
$R^{7'}$ is as defined above and
$P^1$ is a protecting group, or

d) when Y is a group of formula $Y'''$,

oxidizing a corresponding compound of formula DDD

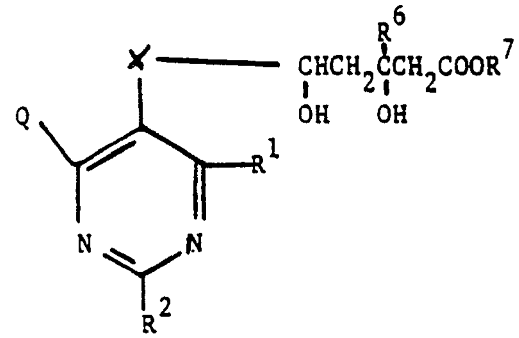

DDD

wherein $R^1$, $R^2$, $R^6$, $R^7$, X and Q are as defined above, and recovering the resultant compounds of formula I in free acid form, or in the form of an ester or δ-lactone thereof, or in salt form as appropriate.

Process variant a) is particularly suited for compounds in ester form.

Process variant b) is particularly suited for compounds in salt form.

Process variant c) is particularly suited for lactones in the 4R,6S or 4R,6R configuration, and for dihydroxy esters and acids in the erythro (3R,5S) configuration and in salt form.

Process variant d) is particularly suited for compounds wherein X is vinylene.

It will readily be appreciated that the various forms of the compounds of formula I may be interconverted as indicated above, whereby lactonisation may only take place when there is a group $Y'$ wherein $R^7$ is hydrogen.

In the same way compounds obtained according to a) to d) may be as appropriate hydrolysed to free acid forms and free acid forms may be esterified or lactonised to produce a desired end-product. The invention thus also provides a process for preparing a compound of formula I which comprises hydrolysing a compound of formula I in ester or lactone form, or esterifying or lactonising a compound of formula I in free acid form, and when a free carboxyl group is present recovering the compound obtained in free acid form or in the form of a salt as appropriate.

Unless otherwise stated reactions are performed in a manner conventional for the type of reaction involved. Molar ratios and reaction times are as a rule conventional and non-critical and are chosen according to principles well established in the art on the basis of reactions and conditions employed.

Process a) (reduction) is a mild reduction and may be accomplished in conventional manner for reducing a ketone to a secondary alcohol. A particularly convenient method of accomplishing process a) is a three step process which involves first treating a compound of formula AAA with a trialkyl borane, (process variant a′), then treating the reaction mixture with sodium borohydride to obtain a complex (process variant a″) and then cleaving the complex by treating the product with methanol (process variant a‴), to obtain the corresponding diol ester compound of formula $Ia^1$.

Process variant a), including variants a′), a″) and a‴) are all preferably carried out under anhydrous conditions. In process variant a′) the alkyl groups of the trialkyl borane may have 2 to 4 carbon atoms and be primary or secondary, e.g. triethyl borane. About 1 to 1.3 moles of the trialkylborane per mole of compound J, especially 1.02:1 to 1.3:1, may be employed in this step. Optionally air may be bubbled through the reaction mixture. The reaction is carried out in an inert medium, e.g. a cyclic ether such as tetrahydrofuran (THF), preferably in a mixture of THF and a $C_{1-4}$alcohol, e.g. methanol, in a ratio of about 3 to 4:1 (v/v) as reported in Tetrahedron Letters 28, 155 (1987), e.g. at about -80° to about +30° C.

Process variant a″) is preferably carried out at reduced temperatures, e.g. about -100° to about -40° C, especially -78° C, and sodium borohydride ($NaBH_4$) added thereto; the medium and essentially anhydrous conditions employed in process variant a′) being maintained. After sufficient reaction time, the temperature is allowed to rise, e.g. to about -20° C, the reaction mixture may be quenched with an aqueous agent, e.g. dilute hydrochloric acid, and the product (complex) recovered and refined if desired, or the reaction product may conveniently be reacted by process variant a‴) in situ.

In process variant a‴) the product of process variant a″) is preferably treated with a large excess of

methanol at moderate temperatures, e.g. + 20° to + 30° C, to obtain the corresponding compound of formula $Ia^1$.

Process a) in general gives a mixture of erythro and threo isomers. Where a high proportion of erythro form is desired, the three-step variant is preferably employed.

The above-described three-step reduction process variant a) is rather stereoselective and yields predominantly the erythro stereoisomer (racemate). If a mixture having a richer portion of threo stereoisomer is desired, then the reduction may be carried out by process variant $a^{IV}$). Process variant $a^{IV}$) may be carried out employing a mild reducing agent, such as sodium borohydride or, preferably, a complex of t-butylamine and borane in an inert organic solvent such as an ether or a lower alkanol, preferably tetrahydrofuran or ethanol, conveniently at a temperature of -10° to +30° C, e.g. at about 0° C, utilizing at least 1, for example 2-4, equivalents of transferable hydride per mole of compound of formula AAA, in an inert atmosphere.

Process b) (hydrolysis) is effected in manner conventional for such reactions, e.g. employing an inorganic hydroxide such as NaOH or KOH with, if desired, subsequent acidification to give the free acid form. Suitable solvents are mixtures of water and water miscible solvents such as lower alkanols, e.g. methanol or ethanol and reaction conveniently takes place at temperatures of from about 0° C to about reflux temperature, preferably about 0° to about 75° C, e.g. about 20° to about 70° C. If it is desired to recover the compound in a salt form corresponding to the cation of the hydroxide employed then slightly less than equivalent amounts of the latter may be employed. It may be carried out in essentially the same manner as the saponification described above for interconverting a compound of formula Ib or $Ia^1$ to a compound of formula $Ia^2$, except that double the equivalents of MOH is required as saponification occurs at both ester sites on compounds of formula BBB.

Process c) (deprotection) is carried out in conventional manner, e.g. by cleavage under mild conditions, such as employing e.g. for removal of a diphenyl-t-butylsilyl group a fluoride reagent, e.g. tetra-n-butylammonium fluoride, in an anhydrous inert organic medium, preferably tetrahydrofuran, containing glacial acetic acid, at temperatures of from about 20° to about 60° C, especially from about 20° to about 25° C. Preferably about 1 to about 10 moles of fluoride per mole protecting group are used with about 1.0 to about 1.5 moles of glacial acetic acid to each mole of fluoride.

Examples of protecting groups are trisubstituted silyl radicals such as diphenyl-t-butylsilyl, tri-isopropylsilyl and dimethyl-t-butylsilyl. Especially preferred is diphenyl-t-butylsilyl.

Process d) (oxidation) is also effected in conventional manner, it may e.g. be carried out, when X is vinylene, using activated $MnO_2$ or 2,3-dichloro-5,6-dicyano-1,4-benzoquinone (DDQ) at a temperature of from about 20° to about 80° C, preferably from about 40° to about 80° C, in an anhydrous inert organic solvent such as an ether solvent, e.g. $(C_2H_5)_2O$, 1,2-diethoxyethane, 1,2-dimethoxyethane, tetrahydrofuran and mixtures thereof or, when X is ethylene, using Swerns reagent (oxalyl chloride + dimethylsulfoxide) with triethylamine in e.g. $CH_2Cl_2$ at from about -60° to about -40° C, preferably about -50° C.

The starting materials may be obtained in conventional manner.

The compounds of formula AAA wherein X is -CH=CH- may e.g. be obtained in accordance with reaction scheme B below, whereby compounds of formula E therein may in turn e.g. be obtained by reaction scheme C below, whereby Et is ethyl, $X''$ is a magnesium halide, e.g. -MgCl or -MgBr, $X^{\circ}$ is a Wittig reagent, and Q, $R^1$, $R^2$ and $R^{7'}$ are as defined above:

Reaction scheme B

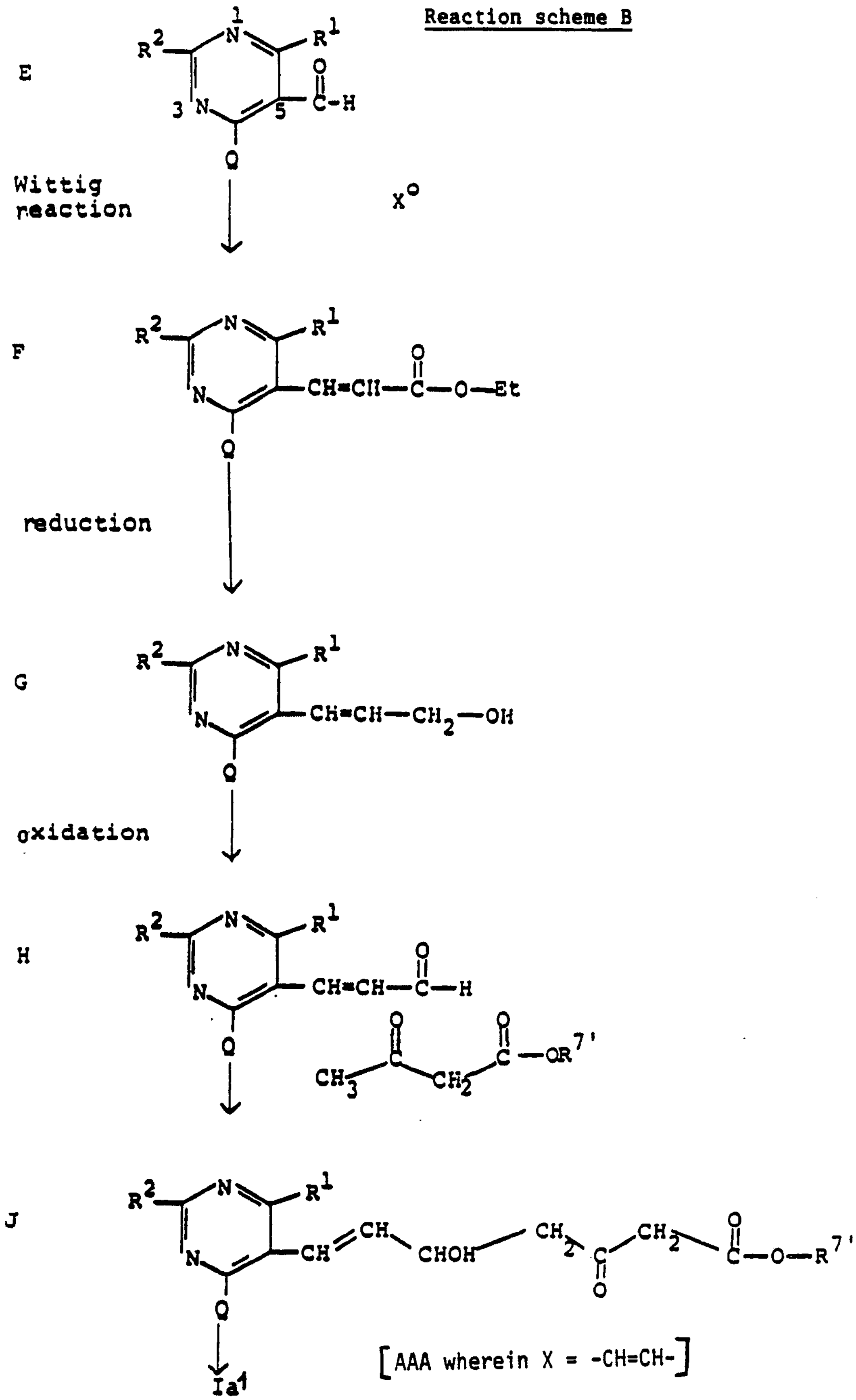

Reaction scheme C

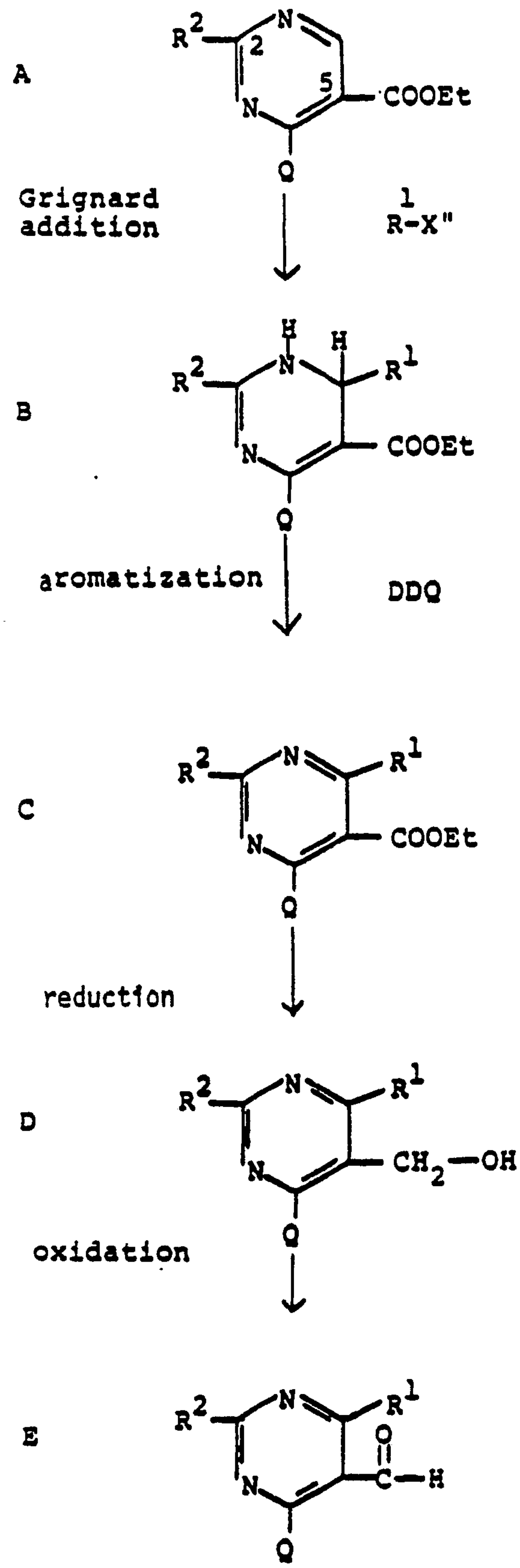

Compounds A in reaction scheme C are known and are obtainable by methods described in the literature, or are obtainable by methods analogous to those described for preparing the known compounds, e.g. as represented in reaction scheme D below, whereby Q, $R^2$ and Et are as defined above:

Reaction scheme D

W

Y

Z

A

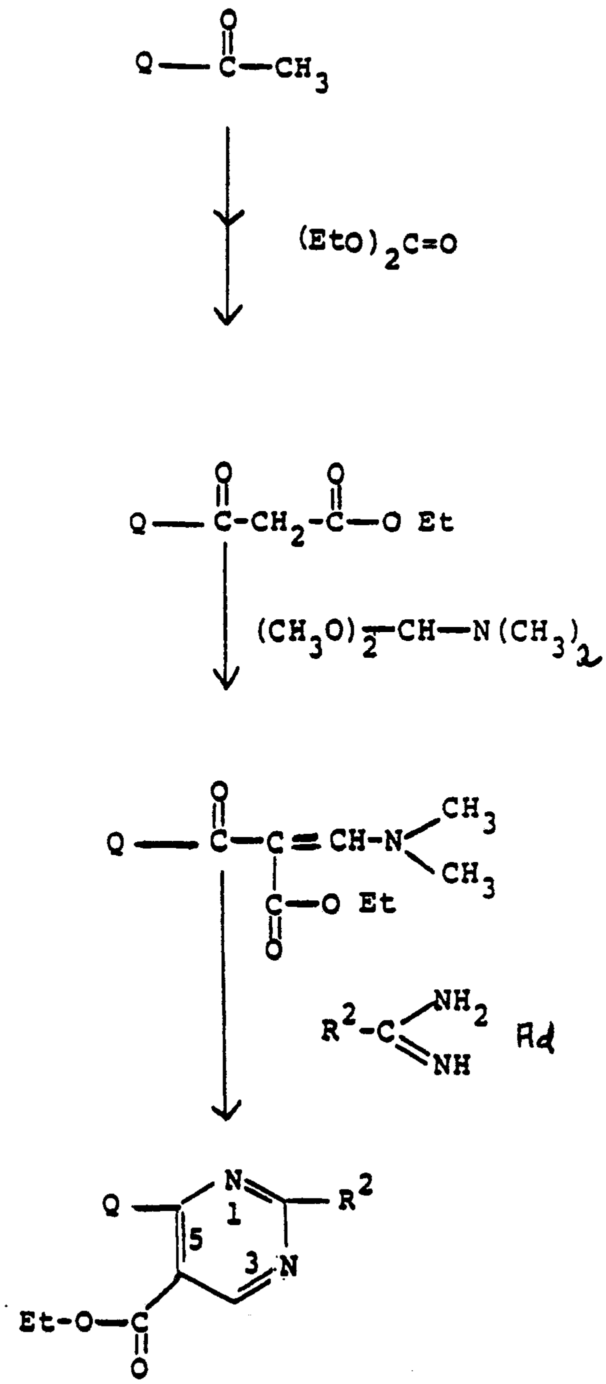


Alternatively, compounds C (intermediates in scheme C) may conveniently be obtained by a series of known reaction steps represented in reaction scheme H, below, whereby Q, $R^1$, $R^2$ and Et are as defined above:

Reaction scheme H

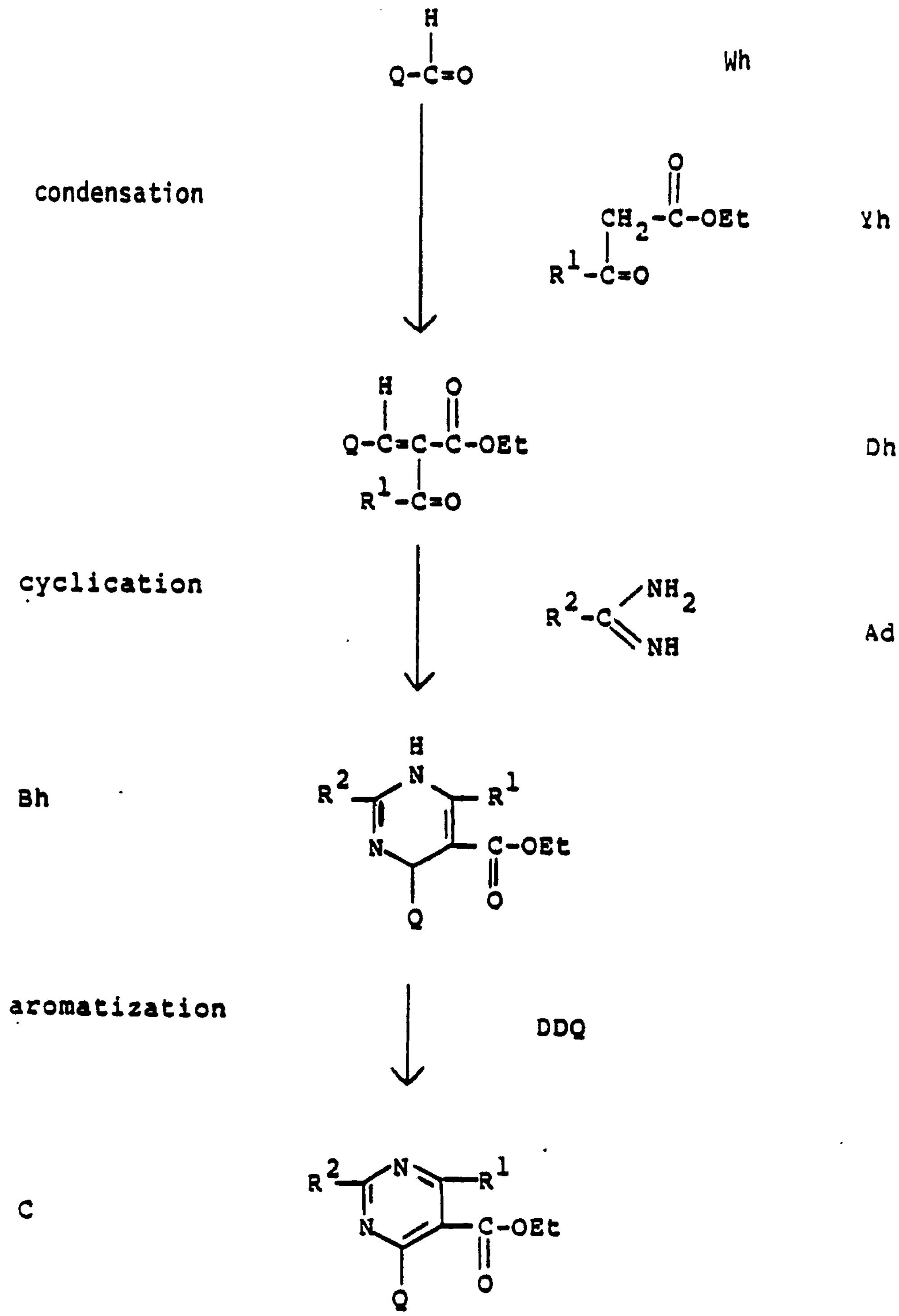


The compounds of formula AAA wherein X is $-CH_2CH_2-$ may e.g. be obtained by a series of reaction steps starting from a compound E as represented in reaction scheme E below, in which $R^a$ is a pyrimidinyl moiety of the formula

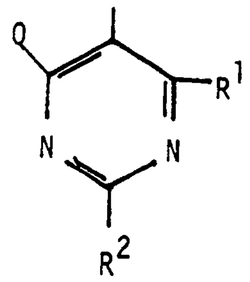

whereby $R^1$, $R^2$, Q and $R^{7'}$ are as defined above:

Reaction scheme E

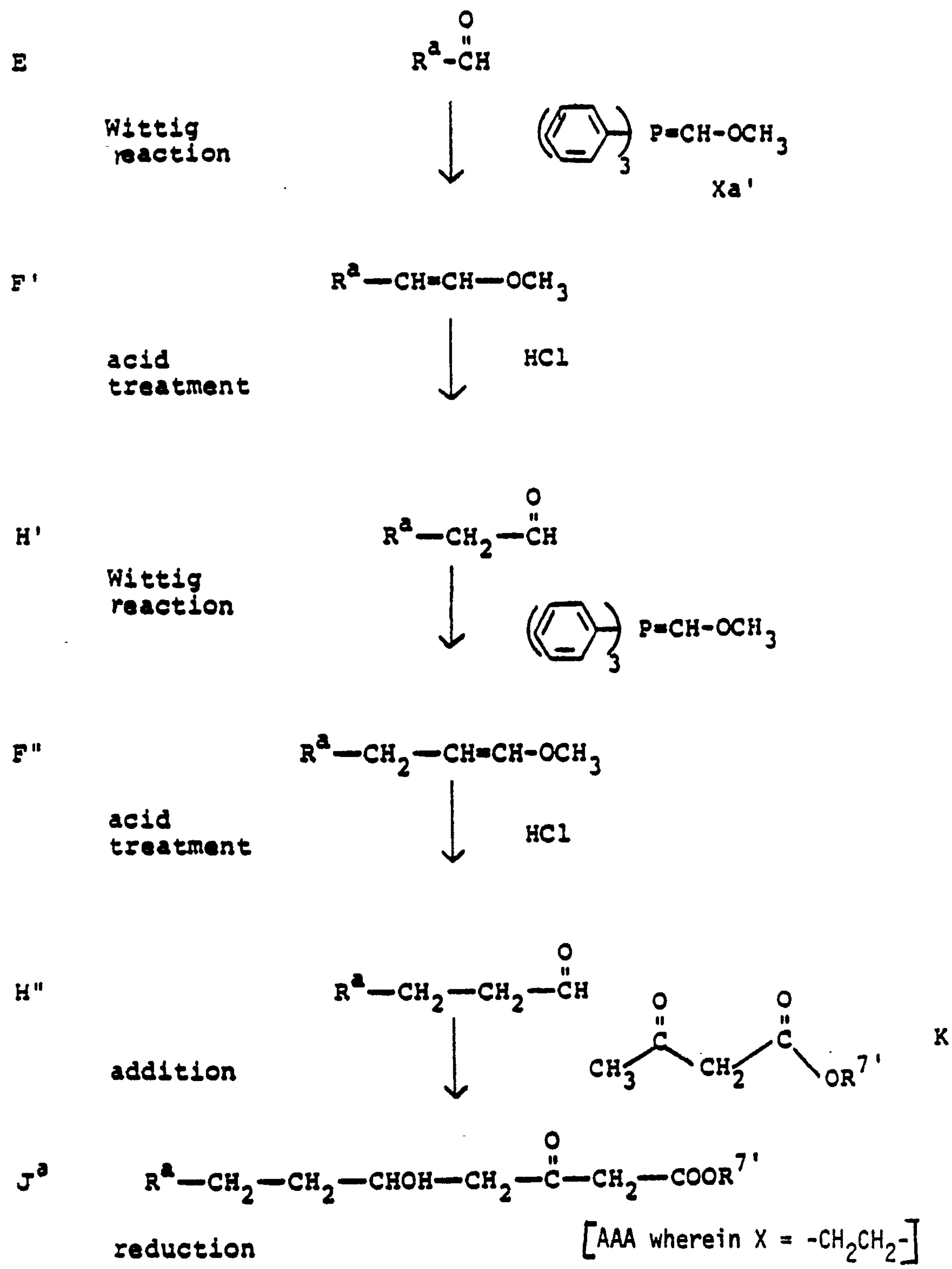


The compounds of formula BBB may e.g. be obtained by employing a series of reaction steps as depicted in reaction scheme F below, wherein $R^b$ is a moiety of formula

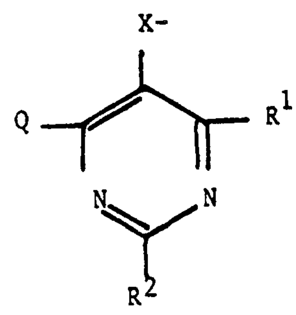

and $R^8$ and $R^9$ are part of an ester-forming group, e.g. $C_{1-3}$alkyl, preferably methyl or ethyl, and D is halo, e.g. bromo or chloro, especially chloro.

## Reaction scheme F

H"

P

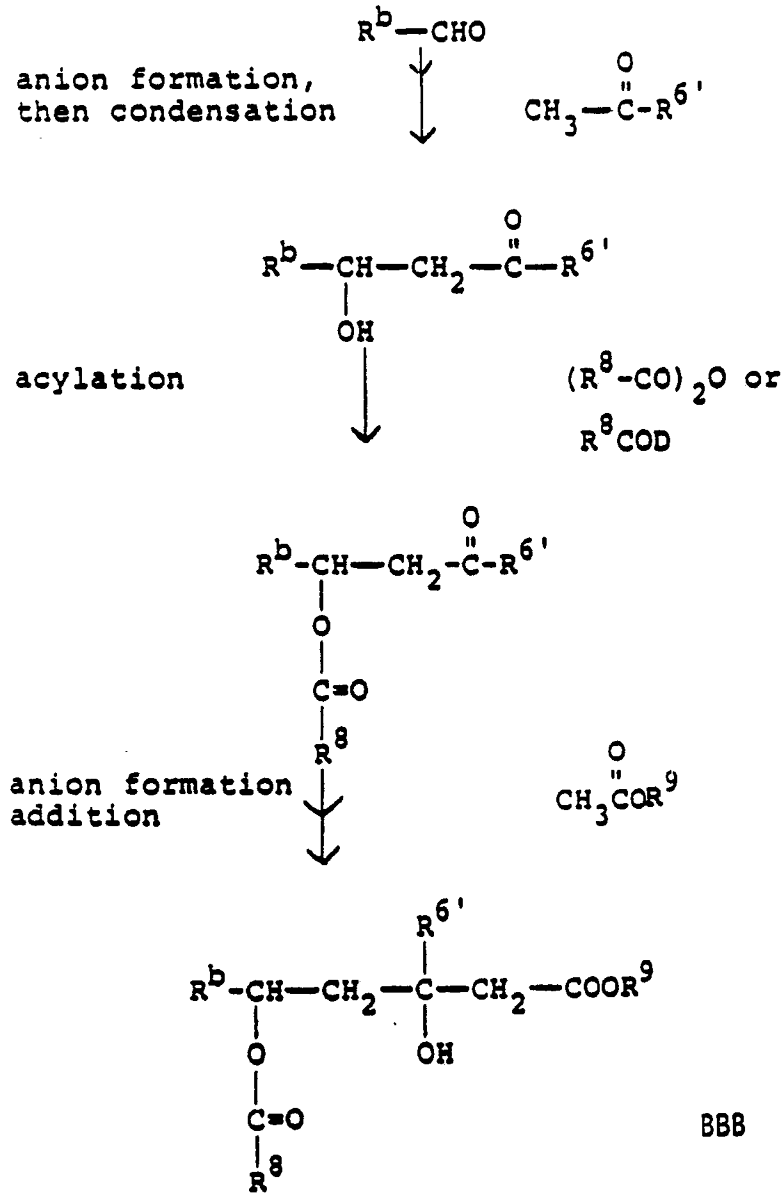


Q

R

S

T

U

BBB

The compounds of formula CCC wherein $Y^{IV}$ is $Y_a^{IV}$ may e.g. be prepared in high optical purity with a Wittig reagent by reaction scheme G below, wherein $R^a$ is as defined above, Ph is phenyl, $P^1$ is a protective group, e.g. a trisubstituted silyl radical in which the substituents are bulky groups, e.g. aryl or tertiary-alkyl, such as diphenyl tert-butyl-silyl, and $P^2$ is n-$(C_{1-4})$alkyl, e.g. methyl:

Reaction scheme G

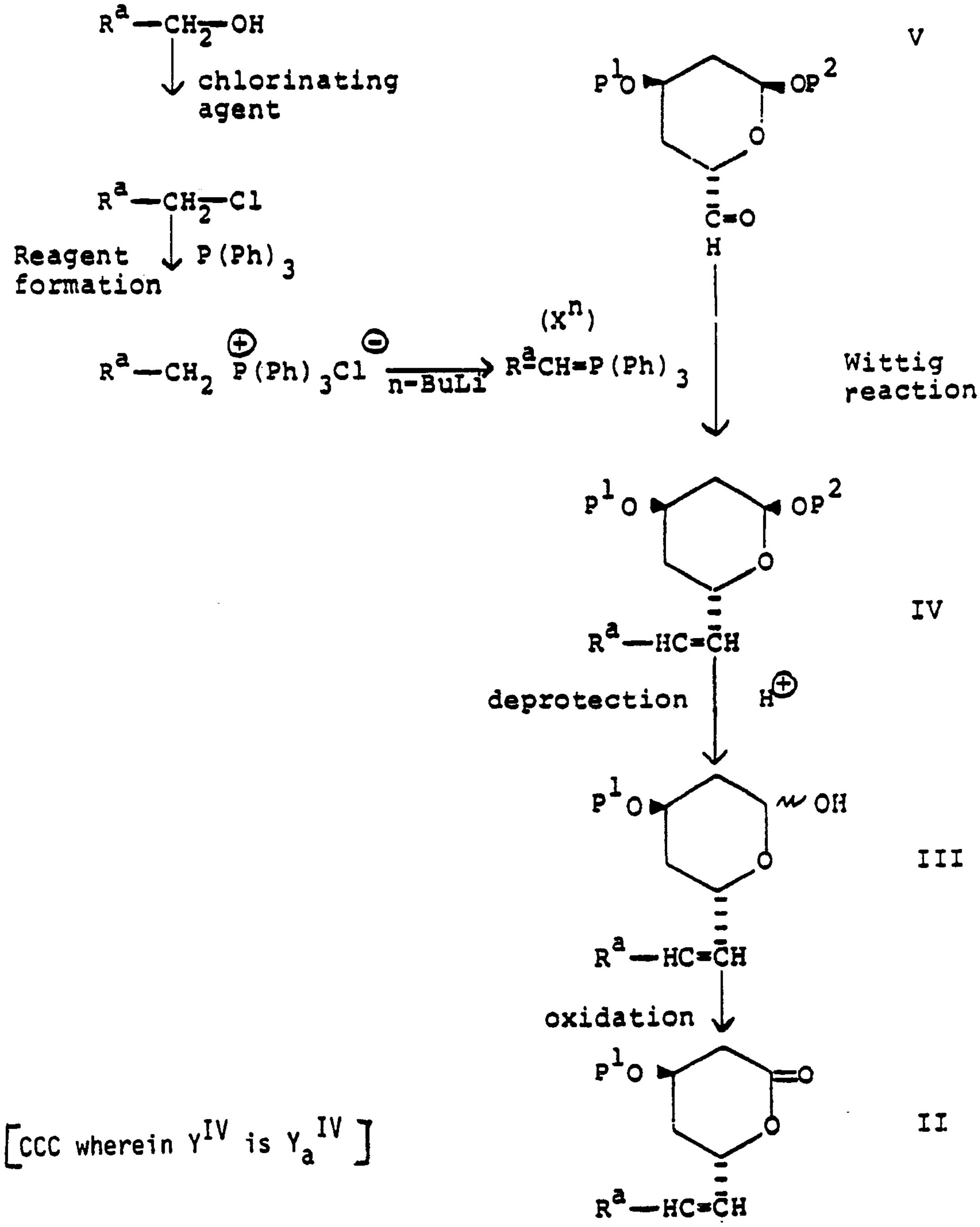


The compounds of formula CCC wherein $Y^{IV}$ is $Y_b^{IV}$ may e.g. be prepared analogously, in high optical purity, carrying out a Wittig reaction between a 3R,5S-dihydroxy-diprotected aldehyde of the formula Hk:

Hk 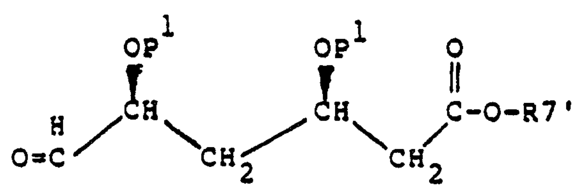

in which $R^{7'}$ and $P^1$ are as defined above, and a Wittig reagent of the formula $X^k$ or $X^n$:

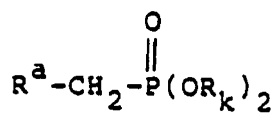

$X^k$

or

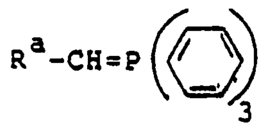

$X^n$

in which $R^a$ is as defined above and $R^k$ is methyl or ethyl, to obtain a corresponding intermediate of the formula IIk:

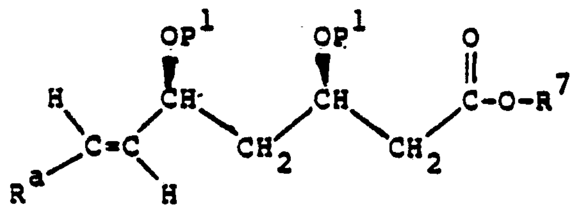

IIk

[CCC wherein $Y^{iv}$ is $Y^{iv}_b$]

in which $R^1$, $R^{7'}$ and $P^1$ are as defined above.

The compounds of formula DDD fall under formula I.

Reagents and starting materials employed in the above-described processes are either known and may be obtained as described in the literature or may be prepared by methods reported in the literature for the preparation of known analogues. Many are commercially available.

The compounds of formula I in free acid form or in physiologically acceptable ester, e.g. δ-lactone form, or in pharmaceutically acceptable salt form as appropriate, hereinafter referred to as "the compounds", exhibit pharmacological activity. They are therefore indicated for use as pharmaceuticals, e.g. for therapy.

In particular the compounds show activity in the following tests:

Test A. In vitro microsomal assay of 3-hydroxy-3-methylglutaryl coenzyme A (HMG-CoA) reductase inhibition: as described in EP 114 027:

The following results are obtained by test A:
Product of Example 11d: $IC_{50}$ = 0.039 µM;
Product of Example 1b): $IC_{50}$ = 0.026 µM;
Compactin; $IC_{50}$ = 1.01 µM;
Mevinolin; $IC_{50}$ = 0.352 µM.

$IC_{50}$ is the concentration of the test substance in the assay system calculated to produce a 50% inhibition of HMG-CoA reductase activity. The tests are run at concentrations of test substance between 0.05 µM and 1000 µM.

Test B. In vivo cholesterol biosynthesis inhibition test: as described in EP 114 027:

The following results are obtained by test B:
Product of Example 11d: $ED_{50}$ = 0.04 mg/kg;
Product of Example 1b): $ED_{50}$ = 0.028 mg/kg;
Compactin; $ED_{50}$ = 3.5 mg/kg;
Mevinolin; $ED_{50}$ = 0.41 mg/kg.

$ED_{50}$ is the dose of the test substance calculated to produce a 50% inhibition of 3β-hydroxysterol synthesis. The studies are run to test doses of between 0.01 mg/kg and 10 mg/kg.

The above test data indicate that the compounds are competitive inhibitors of 3-hydroxy-3-methylglutaryl coenzyme A (HMG-CoA) reductase, the rate limiting enzyme in cholesterol biosynthesis, and, therefore, they are inhibitors of cholesterol biosynthesis. Consequently, they are indicated for use in lowering the blood cholesterol level in animals, e.g. mammals, especially larger primates, and, therefore, indicated for use as hypolipoproteinemic and anti-atherosclerotic agents.

For these indications the exact dosage will of course vary depending upon the compound employed, mode of administration and treatment desired. An indicated suitable daily dosage is in the range from about 1 mg to about 500 mg, preferably from about 1 mg to about 80 mg, conveniently administered, for example, in divided doses 2 to 4 times a day in unit dosage form containing for example from about 0.25 mg to about 250 mg, preferably in unit dosage of from about 0.25 mg to about 25 mg of the compound, or in sustained release form.

The preferred compounds of the invention are the products of Examples 1 and 2 and of Examples 11a-j, especially of Examples 1b) and 2b), particularly of Example 1b).

The compounds may be administered in free acid form, or in physiologically acceptable ester or in δ-lactone form, or in pharmaceutically acceptable salt form as appropriate. Such salts and esters and lactones may be prepared in conventional manner and exhibit the same order of activity as the free acid form.

The present invention also provides a pharmaceutical composition comprising a compound of formula I in any of its forms in association with a pharmaceutically acceptable solid or liquid carrier or diluent. Such compositions may be formulated in conventional manner, e.g. by mixing with a pharmaceutically acceptable carrier or diluent.

The compounds may be administered by any conventional route, in particular enterally, preferably orally, e.g. in the form of tablets or capsules, or parenterally, e.g. in the form of injectable solutions or suspensions.

The invention therefore also concerns a method of treating hyperlipoproteinemia or atherosclerosis by administration of a compound of formula I in any of its forms, as well as such compounds for use as pharmaceuticals, e.g. as hypolipoproteinemic and anti-atherosclerotic agents.

The compounds may be administered alone, or in admixture with a pharmaceutically acceptable diluent or carrier and, optionally, other excipients, and administered orally in such forms as tablets, elixirs, capsules or suspensions or parenterally in such forms as injectable solutions or suspensions. The preferred pharmaceutical compositions from the standpoint of ease of preparation and administration are solid compositions, particularly tablets and hard-filled or liquid-filled capsules.

## EXAMPLES

The following examples are illustrative of the invention. All temperatures are centigrade and room temperature (r.t.) is 20° to 30° unless indicated otherwise.

Evaporations are done under vacuum (i.v.) employing minimal heating. Drying of organic phases is done over anhydrous sodium sulfate unless indicated otherwise. THF is tetrahydrofuran, MTBE is methyl tertiary-butyl ether.

**Example 1: (3R,5S)-[E]-7-[4-(4-fluorophenyl)-6-(1-methylethyl)-2-(dimethylamino)pyrimidin-5-yl]-3,5-dihydroxy-6-heptenoic acid, (1,1-dimethylethyl) ester; and sodium salt**

($R_1$ = isopropyl; $R_2$ = dimethylamino;
Q = 4-fluorophenyl; X = (E)-CH=CH-;
Y = a group Y′ wherein $R^6$ = H, $R^7$ = tert-butyl or Na and the configuration is 3R,5S)
[process variant c) (deprotection) and recovery in salt form]

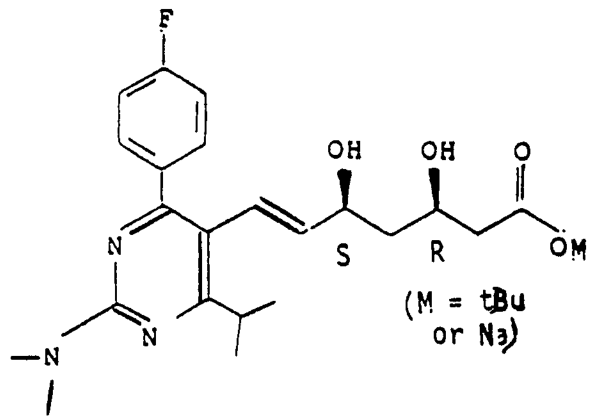

a) Deprotection:

14.2 g of (3R,5S)-[E]-3,5-bis[[(1,1-dimethylethyl)-diphenylsilyl]oxy]-7-[4-(4-fluorophenyl)-6-(1-methylethyl)-2-dimethylamino)pyrimidin-5-yl]-6-heptenoic acid, 1,1-dimethylethyl ester (see below) dissolved in 350 ml $CH_3CN$ is added to a mixture of 47.2 g of tetra n-butylammonium fluoride trihydrate and 350 ml acetonitrile and 9 g (8.6 ml) of glacial acetic acid. This is stirred at 45-50°, and then stirred at 65° for 24 hours under argon. The reaction mixture is poured into 150 ml of saturated sodium chloride solution, 200 ml saturated sodium carbonate solution and 1.35 liters of water (the pH should be approximately 7.5-8.5 after the addition) and the mixture is extracted three times with diethyl ether. The diethyl ether extracts are combined, washed three times with 500 ml portions of water, dried over anhydrous $MgSO_4$, filtered and evaporated at a reduced pressure to yield an oil. The crude product is flash chromatographed on a 230-400 ASTM silica gel using 6:4 mixed hexanes:ethyl acetate as the eluant. A yellow oil is isolated which is triturated to a light yellow powder with hexanes. **(3R,5S)-[E]-7-[4-(4-fluorophenyl)-6-(1-methylethyl)-2-(dimethylamino)pyrimidin-5-yl]-3,5-dihydroxy-6-heptenoic acid, (1,1-dimethylethyl) ester** is obtained (M.P. 114-116°; $[\alpha]_D^{25}$ = +7.7°, $CHCl_3$).

b) Hydrolysis:

12.35 g of the product of step a) above, 26.0 ml of 1N NaOH and 150 ml of ethanol are combined and stirred at room temperature for 3-4 hours. The solvent is rotary evaporated. The residue is treated with approximately 150 ml toluene and the toluene is rotary evaporated. This is repeated, and the final residue is triturated to a light yellow solid with a mixture of hexane-ether. This is filtered and dried to yield **sodium (3R,5S)-[E]-7-[4-(4-fluoro-phenyl)-6-(1-methylethyl)-2-(dimethylamino)pyrimidin-5-yl]-3,5-dihydroxy-6-heptenoate** (M.P. 231-233°; $[\alpha]_D^{25}$ = +33.3°, c = 20.625 mg in 1 ml $H_2O$).

The starting material is obtained as follows:

Step 1: 2-dimethylamino-4-(4-fluorophenyl)-1,4-dihydro-6-(1-methylethyl)-5-pyrimidine-carboxylic acid, ethyl ester:

100 g of (Z)-2-[(4-fluorophenyl)-methylene]-4-methyl-3-oxopentanoic acid ethyl ester, 60 g dimethylguanidine•$H_2SO_4$, and 36 g sodium acetate are combined in 500-800 ml ethanol. The mixture is then refluxed for two days and stirred at room temperature for two days. The resulting composition is rotary evaporated. One liter of water made basic with 2N NaOH is added, then the mixture is extracted 2-3x with ether, and dried over $MgSO_4$. The result is filtered and reduced to yield a yellow solid. To this is added 500 ml hexane, and then it is re-filtered and dried.

Step 2: 2-dimethylamino-4-(4-fluorophenyl)-6-(1-methylethyl)-5-pyrimidinecarboxylic acid, ethyl ester:

A 2 liter 3-necked round bottom flask equipped with a stirrer and gas inlet tubes is charged with 58.5 g

of the product of step 1 and 1.5 liter of dioxane. This is stirred at room temperature and 45 g DDQ (2,3-dichloro-5,6-dicyano-1,4-benzoquinone) is added as a solid in portions. The mixture is stirred at room temperature for 3-4 hours.

The resultant product is added to water containing 10% $Na_2CO_3$, extracted 3x with ether, and dried with $MgSO_4$. The product is filtered and reduced to an orange brown semisolid which is flash chromatographed, eluted with 6/4 mixed hexanes/ethyl acetate and recrystallized from hexane. The final product is recovered in two crops.

Step 3: 2-dimethylamino-4-(4-fluorophenyl)-6-(1-methylethyl)-5-pyrimidinemethanol:

A solution of 52.0 g of the product from step 2 in 500 ml of tetrahydrofuran is stirred at room temperature under argon and 750 ml of 1M DIBAL (diisobutylaluminum hydride) is added dropwise. This is stirred at room temperature overnight.

The reaction is cooled to 0-5° and a total of 500 ml saturated $NH_4Cl$ is added dropwise, while maintaining the temperature less than 20°. A gelatinous solid develops and 500 ml of ether are added. The mixture is filtered, extracted, and dried over $MgSO_4$. The result is reduced to a white solid which is washed in cold hexane (at -78°). The product is recovered in three crops.

Step 4: [[4-(4-fluorophenyl)-2-dimethylamino-6-(1-methylethyl)pyrimidin-5-yl]methyl]phosphonic acid, dimethyl ester:

8.09 g of the product from step 3 in $CH_2Cl_2$ is added to 13.5 ml $SOCl_2$ and refluxed for 4-5 hours. This is rotary evaporated to remove excess $SOCl_2$ and the $CH_2Cl_2$, then treated twice with toluene and then rotary evaporated again. 10-15 ml of $(MeO)_3P$ is added carefully to the residue and refluxed overnight. The resultant product is cooled and rotary evaporated to remove excess $(MeO)_3P$ and a yellow oil is isolated. This is flash chromatographed, eluting with 5/5 methyl t-butylether/ethyl acetate. A clear oil is isolated which dries to a white solid.

Step 5: (3R,5S)-[E]-3,5-bis[[(1,1-dimethylethyl)diphenylsilyl]oxy]-7-[4-(4-fluorophenyl)-6-(1-methylethyl)-2-(dimethylamino)pyrimidin-5-yl]-6-heptenoic acid, 1,1-dimethylethyl ester:

19.2 ml of 1.6M n-butyllithium/hexane is added rapidly dropwise to a solution of 11.7 g of the product of step 4 in 100 ml of dry THF at -78°. The resultant solution is stirred for 10 minutes and a solution of 28 g of 1,1-dimethylethyl-(3R,5S)-3,5-di-[(1′,1′-dimethylethyl)diphenylsilyloxy]-6-oxohexanoate (see below, compound IX) in approximately 65 ml of dry THF is added rapidly dropwise with stirring. The resulting solution is stirred for 15 minutes at -78° and 19.2 ml of 1.5 M lithium diisopropylamide bis (tetrahydrofuran) complex/cyclohexane is added with a syringe with stirring at -78°, then the reaction mixture is allowed to warm to 20-25° and stirred for 30 minutes. The reaction mixture is stirred under argon throughout. The reaction mixture is poured into a mixture of 800 ml of water and 150 ml of saturated ammonium chloride solution, and the mixture is extracted three times with diethyl ether. The diethyl ether extracts are combined, dried over anhydrous magnesium sulfate, filtered and evaporated at reduced pressure to obtain a yellow oil. This is flash chromatographed on 230-400 mesh ASTM silica gel utilizing 8/2 mixed hexanes/methyl t-butyl ether as the eluant, and the fractions containing the product (as indicated by thin layer chromatography) are combined and evaporated at reduced pressure to obtain the product as a yellow resin.

The 6-hexanoate (compound IX) used in step 5 is obtained as follows:

Step 1′: dimethyl (S)-hydroxybutanedioate (compound II):

(a) 750 g of acetyl chloride is added over a period of 30 minutes to 13.5 l of methanol stirred at -5° to 0° while maintaining an internal temperature of 0° to 2° (the addition being very exothermic), the reaction mixture is stirred at 0° for 1 hour, 1.76 kg of (S)-hydroxybutanedioic acid (= compound I) is added over a period of 30 minutes while maintaining an internal temperature of 0° to 2° (the addition being slightly exothermic), and the reaction mixture is stirred at 0° to 2° for 2 hours and at 20° to 25° for 16

hours, the reaction mixture being stirred under nitrogen throughout. 898 g. of anhydrous sodium carbonate is added over a period of 15 minutes while maintaining an internal temperature of 20° to 25° to adjust the pH to 7-8 (the addition being slightly exothermic), and the reaction mixture is stirred at 20° to 25° for 30 minutes and filtered. The filter cake is washed twice with 500 ml portions of methanol, the filtrate and washings are combined, and the solution is evaporated at 25-30 mm Hg while maintaining an internal temperature of 35° to 40° to a volume of about 2.5 l and filtered. The filter cake is washed with 500 ml of methanol, the filtrate and washings are combined, and the solvent is distilled at 25-35 mm Hg and an internal temperature of 40° to obtain the crude product.

(b) A solution of 300 g of crude product from part (a) of this step in 300 ml of methylene chloride is added to a slurry of 300 g of 70-230 mesh ASTM silica gel in 1.4 l of methylene chloride. The methylene chloride is distilled at 30-40 mm Hg and an external temperature of 50° to obtain the partially purified product which is vacuum distilled at 0.6 mm Hg and an internal temperature of 96° to 98° to obtain the 96.7% pure product (B.P. 90° to 92°/1.5-2 mm Hg; B.P. 78° to 80°/0.6 mm Hg).

(c) The remainder of the crude product is similarly purified.

<u>Step 2′</u>: methyl (S)-3,4-dihydroxybutanoate (compound III):

7.5 g of sodium borohydride is added in one portion to a mixture of 523 ml of 10M borane-dimethylsulfide complex and l.8 l of dry tetrahydrofuran stirred at 20° to 25°, the reaction mixture is stirred at 20° to 25° for 30 minutes, a solution of 744 g of 94% pure compound II in 1.2 l of dry tetrahydrofuran is added over a period of 1 hour while maintaining with external cooling at 3° to 5° an internal temperature of 20° to 25° (the reaction is very exothermic, and hydrogen is evolved), and, after the exotherm stops, the reaction mixture is stirred at 20° to 25° for 1 hour and cooled to 20°, the reaction mixture being stirred under nitrogen throughout. 1.8 l of methanol is slowly added while maintaining an internal temperature of 20° to 25° (the first third of the addition is very exothermic and foaming occurs). The reaction mixture is stirred for 30 minutes while maintaining an internal temperature of 20° to 25°, the solvent is distilled at 20-30 mm Hg and an external temperature of 40° to 45°, 500 ml of toluene is added, the solvent is distilled at 20-30 mm Hg and an external temperature of 40° to 45°, an additional 500 ml of toluene is added, and the solvent is distilled at 20-30 mm Hg and an external temperature of 40° to 45° to obtain the crude product as a very thick stirrable oil. The entire reaction and the solvent distillations must be vented through bleach to trap any escaping dimethylsulfide.

<u>Step 3′</u>: methyl (S)-3-hydroxy-4-(triphenylmethoxy)butanoate (compound IV):

(a) A solution of 1.82 kg of triphenylmethyl chloride in 2.5 l of methylene chloride is added over a period of 12 minutes to a mixture of 716 g of 81.7% pure compound III, 880 g of triethylamine and 4.9 l of methylene chloride stirred at 15° while maintaining an internal temperature of 15° to 20° (the addition being exothermic), and the reaction mixture is stirred at 23° for 16 hours, stirred at an internal temperature of 38° to 40° for 15 minutes (during which gas evolves) and cooled to 25°, the reaction mixture being maintained under nitrogen throughout. 7.4 l of water is added, the bottom organic layer is separated, the aqueous layer is extracted with 500 ml of methylene chloride, the methylene chloride extract is combined with the bottom organic layer, and the combined organic layers are washed successively with 7.4 l of water, 7.4 l of saturated sodium bicarbonate solution and 3.7 l of water and filtered to remove any insolubles. The solvent is distilled at 20-30 mm Hg and a maximum external temperature of 50° to 60° to obtain a thick dark stirrable oil. 2.5 l of methanol is added with stirring at 50°, and the mixture is stirred at 50° for 5 minutes, cooled to 20° and filtered. The filter cake is washed with 1 l of methanol, the filtrate and washing are combined, and the solvent is distilled at 20-30 mm Hg and an external temperature of about 50° to obtain the crude product as a thick stirrable brown oil.

(b) A 90 cm x 13 cm chromatographic column is charged with 7.2 l of 1:2 ethyl acetate/mixed hexanes, 450 g of sea sand is added, a slurry of 4 kg of 70-230 mesh A.S.T.M. silica gel in 12 l of 1:2 ethyl acetate/mixed hexanes is added while draining some of the solvent from the bottom of the column, sufficient solvent to lower the liquid level to the top of the silica gel is drained from the column, a solution of 1.0 kg of the crude product of part (a) of this step in 1.0 l of methylene chloride is added, sufficient solvent to lower the liquid level to the top of the silica gel is drained from the column, the column is eluted with 18 l of 1:2 ethyl acetate/mixed hexanes to obtain six fractions, and the column is eluted with 12 l of 1:1 ethyl acetate/mixed hexanes to obtain eight fractions containing relatively pure product. These eight fractions are combined, the solvent is distilled at 20-30 mm Hg and 60° to obtain a thick stirrable oil, 1 l of isopropanol

is added, the solvent is distilled at 20-30 mm Hg and a maximum internal temperature of 60°, an additional 1 l of isopropanol is added, the solvent is distilled at 20-30 mm Hg and a maximum internal temperature of 60°, and the residue is added to 2 l of isopropanol. The resulting solution is cooled to 0° to 5° and maintained at this temperature for 3-4 hours. The solid is collected by filtration, washed twice with 250 ml portions of cold (0° to 5°) isopropanol and vacuum dried at 50° to 60° to constant weight to obtain the 94.9% pure product [M.P. 80° to 82°; $[\alpha]^{25}_{589}$ = -5.52° (c = 1.5580, $CH_2Cl_2$]. All chromatography fractions and mother liquors containing impure product may be combined and rechromatographed, etc. to obtain additional product.

(c) The balance of the crude product is similarly purified.

Step 4′: 1,1-dimethylethyl (S)-5-hydroxy-3-oxo-6-(triphenylmethoxy)hexanoate (compound V):

1.98 l of 1.6M n-butyllithium/hexane is added over a 30 minute period to a mixture of 321 g of diisopropylamine and 1.5 l of dry tetrahydrofuran stirred at -50° to -40°, during which the internal temperature rises to -5° to 0°, the pale yellow solution is stirred at -5° to 0° for 30 minutes and cooled to -65°, 367 g of t-butyl acetate is added over a period of 40 minutes while maintaining an internal temperature of -63° to -60° (the addition being exothermic), the reaction mixture is stirred at -62° to -60° for 40 minutes, a solution of 300 g of more than 95% pure compound IV in 1.35 l of dry tetrahydrofuran is added over a period of 25 minutes while maintaining an internal temperature of -62° to -58° (the addition being exothermic), and the reaction mixture is stirred at -62° to -58° for 1 hour, warmed to -5° to 0° over a period of 45 minutes and stirred at -5° to 0° for 30 minutes, the reaction mixture being stirred under nitrogen throughout. 1.8 l of saturated ammonium chloride solution is added over a period of 3 minutes while an internal temperature of below 15° (the addition being exothermic), the mixture is stirred at 10° to 15° for 10 minutes, 2.1 l of toluene is added, and the top organic layer is separated, washed three times with 1.5 l portions of water and, if any insolubles are present, filtered. The solvent is distilled at 20 to 30 mm Hg and an internal temperature of 50° to 60° to obtain the crude (87.1 % pure) product as a very thick yellow oil that is stirrable at 50°.

Step 5′: 1,1-Dimethylethyl (3R,5S)-3,5-dihydroxy-6-(triphenylmethoxy)hexanoate (compound VI):

A mixture of 715 ml of 1M triethylborane/tetrahydrofuran, 4.76 l of dry tetrahydrofuran and 1.44 l of methanol is stirred at 18° to 22° for 45 minutes and cooled to -75°, a solution of 254.4 g of crude (90% pure) compound V in 960 ml of dry tetrahydrofuran is added over a period of 15 minutes while maintaining an internal temperature of -75° to -72°, the reaction mixture is stirred at -76° to -75° for 1 hour, 25 g of sodium borohydride is added in portions over a period of 15 minutes while maintaining an internal temperature of -76° to -72°, and the reaction mixture is stirred at -77° to -76° for 4.5 hours, the reaction mixture being stirred under nitrogen throughout. 756 ml of saturated ammonium chloride solution is slowly added over a period of 25 minutes while maintaining an internal temperature of -76° to -67° (the addition is exothermic, and some foaming occurs), the mixture is stirred at -68° to -67° for 30 minutes and warmed to 10° to 15° over a period of 30 minutes, 360 ml of water is added, the mixture is extracted with 3.6 l of ethyl acetate, the top organic layer is separated and washed successively with 750 ml of a 1:1 mixture of water and saturated sodium chloride solution and 750 ml of saturated sodium chloride solution, and the solvent is distilled at 20-30 mm Hg and a maximum external temperature of 50°. 180 ml of ethyl acetate is added, the solvent is distilled at 20-30 mm Hg and a maximum external temperature of 50°, and these two steps are repeated once. 2.91 l of ethyl acetate is added to the obtained thick oil (the cyclic boronate), 290 ml of 30% hydrogen peroxide solution is slowly added over a period of 20-30 minutes while maintaining an internal temperature of 20° to 25° (the addition initially being exothermic), and the reaction mixture is stirred at 20° to 25° for 2.5 hours and added to 360 ml of water. The top organic layer is separated, washed three times (for 10-15 minutes each time) with 480 ml portions of cold (0° to 5°) 10% sodium sulfite solution (until the organic layer is free of peroxide) while maintaining an internal temperature of 25° and washed successively with 480 ml of saturated sodium bicarbonate solution, 480 ml of water (any emulsion that forms will separate within about an hour) and 480 ml of saturated sodium chloride solution. The top organic layer is separated, and the solvent is distilled at 20-30 mm Hg and a maximum external temperature of 50° to 60°. 326 ml of methanol is added, the solvent is distilled at 20-25 mm Hg and an internal temperature of 50° to 60°, and these two steps are repeated four more times. 100 ml of toluene is added, the solvent is distilled at 20-30 mm Hg and an internal temperature of 60°, and these two steps are repeated three more times. The obtained oil is heated for an additional 3-4 hours at 20-30 mm Hg and 50°

to 60° to obtain the crude (66.8% pure) product (the ratio of the 3R,5S isomer to the 3S,5S isomer is about 69:1 although in other batches it is as low as about 23:1.)

Step 6′: 1,1-Dimethylethyl (3R,5S)-3,5-di-(1′,1′-dimethylethyl)diphenylsilyloxy]-6-(triphenylmethoxy)-hexanoate (compound VII):

132.5 g of imidazole is added to a mixture of 267 g of crude (55.8% pure) compound VI and 900 ml of N,N-dimethylformamide while maintaining an internal temperature of 23° to 25°, the mixture is heated to 70° to 72°, 235.8 g of t-butyldiphenylchlorosilane is added over a period of 2 minutes, and the reaction mixture is stirred at 70° to 72° for 18 hours and cooled to 10°, the reaction mixture being stirred under nitrogen throughout. The reaction mixture is added to a mixture of 2.5 l of hexane and 1.1 l of water, the mixture is stirred for 5 minutes, the organic top layer is separated and washed twice with 1.1 l portions of water, and the solvent is distilled at 20-30 mm Hg and an external temperature of 40° to 60° to obtain the crude (62.8% pure) product.

Step 7′: 1,1-Dimethylethyl (3R,5S)-3,5-di[(1′,1′-dimethylethyl)diphenylsilyloxy]-6-hydroxyhexanoate (compound VIII):

(a) A cold solution prepared from 960 g of water, 600 g of ice and 740 g of trifluoroacetic acid is added over a period of 2 minutes to a solution of 432 g of crude compound VII in 3.4 l of methylene chloride while maintaining a maximum internal temperature of 25°, and the reaction mixture is stirred at 22° to 26° for 2.5 hours, the reaction mixture being stirred under nitrogen throughout. The organic bottom layer is separated and washed successively with 1.1 l of saturated sodium bicarbonate solution and 1.0 l of water, and the solvent is distilled at 20-30 mm Hg and a maximum external temperature of 60°. 100 ml of mixed hexanes is added to the residue, the solvent is distilled at 20-30 mm Hg and a maximum internal temperature of 60°, and these two steps are repeated once, the distillation being continued until no more solvent distills off. 1.1 l of mixed hexanes is added to the obtained thick semi-solid, the mixture is heated at 40° to 45° for 10-15 minutes, cooled to 0° to 5° and maintained at 0° to 5° for 10 minutes, and the solid is collected by filtration and washed twice with 100 ml portions of cold (10°) mixed hexanes. The filtrate and hexane washings are combined, and the solvent is distilled at 20-30 mm Hg and an external temperature of 40° to 60° to obtain the crude product as an oil.

(b) The crude product of part (a) is purified by HPLC using a Kiloprep 250 Millipore Process HPLC system with a reverse phase Kiloprep 250 $C_{18}$ cartridge (80 microns) and a 200 nm detector. The column is flashed with methylene chloride until it is decontaminated (as indicated by the UV detector) and flushed with methanol for about 10 minutes and then with 9:1 methanol/water for about 6 minutes at a flow rate of 0.5 l/min. A solution of the crude product of part (a) of this step in a mixture of 393 ml of methanol and 22 ml of water is placed on the column, and the column is eluted with 9:1 methanol/water for about 22 minutes, 11 minutes and 3 minutes to obtain fractions 1-3 and with methanol for about 4 minutes and 7 minutes to obtain fractions 4 and 5, the flow rate being 0.5 l/min. Fractions 4 and 5 are combined, the solvent is distilled at 20-40 mm Hg and a maximum internal temperature of 40°, 250 ml of methylene chloride is added to the residue, the mixture is stirred at 30° for 5 minutes, the organic bottom layer is separated, and the solvent is distilled at 20-30 mm Hg and a maximum internal temperature of 40° to 45°. 100 ml of n-heptane is added, the solvent is distilled at 20-30 mm Hg and 40° to 45°, and these two steps are repeated once. 460 ml of n-heptane is added to the residue, and the mixture is heated to 80° to 90° to dissolve the oil, allowed to cool to 20° to 25° with stirring until solid forms and maintained at 10° for 2-3 hours. The resulting white solid is collected by filtration, washed twice with 50 ml portions of cold (10°) n-heptane and vacuum dried to constant weight at 50° for 15 hours to obtain the 95.5% pure product as a white solid [M.P. 79° to 80°, $[\alpha]^{25}_{589}$ = +8.35° (c = 5.0, $CH_2Cl_2$)] (the ratio of the 3R,5S isomer to the 3S,5S isomer is about 76:1).

Step 8′: 1,1-Dimethylethyl (3R,5S)-3,5-di-[(1′,1′-dimethylethyl)-diphenylsilyloxy]-6-oxohexanoate (compound IX):

150 g (0.70 mole) of pyridinium chlorochromate is added over a period of 3 minutes to a mixture of 200 g of 96.6% pure compound VIII, 2 l of methylene chloride and 400 g of 4Å powdered molecular sieves stirred at 20° while maintaining an internal temperature of 20° to 22° (the addition being slightly

exothermic), and the reaction mixture is vigorously stirred for 1 hour at 22° to 25°, the reaction mixture being stirred under nitrogen throughout. 3.32 l of n-heptane is added, and the mixture is stirred at 23° for 5 minutes and filtered through 250 g of Celite[R] filter aid pre-wet with n-heptane. The filter cake is washed twice with 200 ml portions of n-heptane, the filtrate and the n-heptane washings are combined, and the solvent is distilled at 20-30 mm Hg and a maximum external temperature of 45° to 50°. 100 ml of n-heptane is added, the solvent is distilled at 20-30 mm Hg and a maximum external temperature of 45° to 50°, and this is repeated once. The resulting oil is dissolved in 400 ml of 1:12 ethyl acetate/mixed hexanes and chromatographed on 420 g of 70-230 mesh ASTM silica gel topped with about 2.5 cm of sea sand utilizing a column having a 7 cm inside diameter and 4 l of 1:12 ethyl acetate/mixed hexanes as the eluant. The solvent is distilled at 20-30 mm Hg and an external temperature of 50° to 55°. 100 ml of n-heptane is added, the solvent is distilled at 20-30 mm Hg and an external temperature of 50° to 55°, this is repeated once, and the residue is maintained at this pressure and temperature for 2 hours. 335 ml of mixed hexanes is added at 40° to 50° to the obtained thick oil and the mixture is cooled to -35° to -30°, seeded and maintained at -35° to -30° for 1 hour. The resulting white solid is collected by filtration, washed twice with 50 ml portions of cold (-25° to -20°) mixed hexanes and dried to constant weight at 20-30 mm Hg and 45° to 50° for 24 hours to obtain the 99% pure product [M.P. 81°; $[\alpha]^{25}_{589}$ = +5.21° (c = 5.0, $CH_2Cl_2$)] (the ratio of the 3R, 5S isomer to the 3S, 5S isomer is about 99:1).

**Example 2: (3R,5S)-[E]-7-[4-(4-fluorophenyl)-6-(1-methylethyl)-2-(4-morpholinyl)pyrimidin-5-yl]-3,5-dihydroxy-6-heptenoic acid, (1,1-dimethylethyl) ester; and sodium salt**

($R^1$ = isopropyl; $R^2$ = 4-morpholinyl; Q = 4-fluorophenyl; X = (E)-CH=CH-; Y = a group Y′ wherein $R^6$ = H, $R^7$ = tert-butyl or Na and the configuration is 3R,5S)
[process variant c) (deprotection and interconversion to or recovery in salt form]

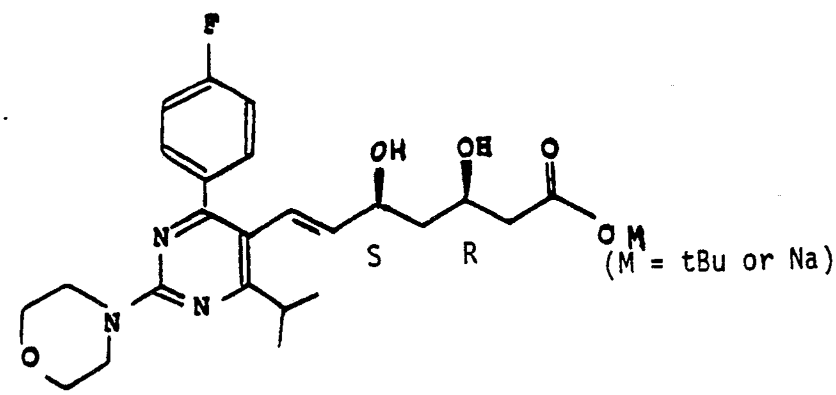

a) Deprotection:

0.80 g of (3R,5S)-[E]-3,5-bis[[(1,1-dimethylethyl)-diphenylsilyl]oxy]-7-[4-(4-fluorophenyl)-6-(1-methylethyl)-2-(4-morpholinyl)pyrimidin-5-yl]-6-heptenoic acid, 1,1-dimethylethyl ester (see below) is reacted in a manner analogous to Example 1) a) to yield **(3R,5S)-[E]-7-[4-(4-fluorophenyl)-6-[(1-methylethyl)-2-(4-morpholinyl)pyrimidin-5-yl]-3,5-dihydroxy-6-heptenoic acid, (1,1-dimethylethyl) ester** (M.P. 134-136°).

b) Hydrolysis

0.127 g of the product of deprotection a) above is treated in a manner analogous to Example 1 b) to yield **(3R,5S)-[E]-7-[4-(4-fluorophenyl-6-(1-methylethyl)-2-(4-morpholinyl)pyrimidin-5-yl]-3,5-dihydroxy-6-heptenoic acid, sodium salt** (yellow-white solid; M.P. 204-208° [dec.]).

The starting material is obtained as follows:

Step 1:

[[4-(4-fluorophenyl)-6-(1-methylethyl)-2-(4-morpholinylpyrimidin-5-yl]methyl]phosphonic acid, dimethyl ester [M.P. 114.5 - 115.5°; white powder] is obtained starting from 4-(4-fluorophenyl)-6-(1-methylethyl)-2-(4-morpholinyl)-5-pyrimidinemethanol in a manner analogous to Example 1, step 4, using the corresponding product from step 3 having 4-morpholino in place of dimethylamino.

Step 2:

(3R,5S)-(E)-3,5-bis[[(1,1-dimethylethyl)diphenylsilyl]oxy]-7-[4-(4-fluorophenyl)-6-(1-methylethyl)-2-(4-morpholinyl)pyrimidin-5-yl]-6-heptenoic acid, 1,1-dimethylethyl ester (yellow resin, 99% pure) is obtained starting from the product of step 1 above, in a manner analogous to Example 1, step 5.

**Example 3: [R*S*]-[E]-7-[4-(4-fluorophenyl)-6-(1-methylethyl)-2-phenylpyrimidin-5-yl]-3,5-dihydroxy-6-heptenoic acid, erythro methyl ester; sodium salt; and free acid**

($R^1$ = isopropyl; $R^2$ = phenyl; Q = 4-fluorophenyl; X = (E)-CH=CH-; Y = a group Y′ wherein $R^6$ = H, $R^7$ = methyl, Na or H and the configuration is predominantly erythro [racemate])
[process variant a) (reduction) and interconversion to or recovery in free and salt form]

a) Reduction:

To a solution of 0,90 g (E)-methyl-7-[2-phenyl-4-(p-fluorophenyl)-6-isopropylpyrimidin-5-yl]-3-keto-5-hydroxy-6-heptenoate (see below) in 20 ml dry THF is added dropwise 2.34 ml of triethylborane (1 M solution), 4 ml of air is then bubbled through the solution. The reaction mixture is then cooled to -78° and to it is added 0.11 g sodium borohydride. The reaction mixture is stirred at -78° for 18 hours. Thereafter it is allowed to warm up to -20°, quenched with dilute hydrochloric acid and extracted several times with ether. After the work up solvent is removed in vacuo to dryness to obtain crude product as a residue.

The residue is dissolved in 25 ml dry methanol and stirred for 1 hour. Solvent is then removed in vacuo, the residue taken up in ether, and the ether layer, after washing with water, saturated brine, and drying, is filtered and evaporated i.v. to dryness. The residue is chromatographed over silica gel using hexane/methyl-t butyl ether as the solvent. The desired fractions are collected, evaporated i.v. to dryness and crystallized with hexane/MTBE to give the **title product in methyl ester form** [M.P. 97-101°] (93 % pure erythro form).

b) Hydrolysis:

To a solution of 0.54 g of the product of reduction a) above in 8 ml methanol is added at room temperature 1.28 ml of 1N sodium hydroxide solution. After stirring the reaction mixture at room temperature for 2 hours, the solvent is removed in vacuo, the residue (sodium salt) is treated with water and then with dilute hydrochloric acid (added to a pH of approximately 2) and extracted several times with ether. The combined ether extracts, after washing with water, saturated brine and drying, are filtered and evaporated in vacuo to dryness. The residue on trituration with hexane/MTBE gives the **title product in free acid form** - (M.P. 57-63°). It contains predominantly the erythro form, with a little threo form.

The starting material is obtained as follows:

Step 1: 2-phenyl-4-(4-fluorophenyl)-4-carbethoxy-6-isopropyl-1,6-dihydropyrimidine (a compound B):

To a solution of 4.0 g of 4.0 g 2-phenyl-4-(4-fluorophenyl)-5-carbethoxy-pyrimidine in 50 ml of dry THF is added dropwise 6.8 ml of 2M isopropyl-magnesium chloride solution in dry THF. After stirring at room temperature for 3 hours the reaction mixture is poured into ice-water and extracted several times with chloroform. The combined organic extracts, after washing with water, saturated brine and drying are filtered and evaporated i.v. to dryness. The residue is chromatographed over silica gel with chloroform as the eluent. The desired fractions are combined and evaporated i.v. and the residue crystallized from

ethanol/water to give the product [M.P. 145-147°]. This product may also be called 4-(4-fluorophenyl)-1,6-dihydro-6-(1-methylethyl)-2-phenyl-5-pyrimidinecarboxylic acid ethyl ester.

Step 2: 2-phenyl-4-(4-fluorophenyl)-5-carbethoxy-6-isopropylpyrimidine (a compound C):

To a solution of 2.5 g of the product of step 1 above in 25 ml dry THF, is added dropwise 1.55 g DDQ in 50 ml THF. After stirring the reaction mixture a few minutes, on completion of the addition of DDQ solution, solvent is removed in vacuo. From the residue the product of this step is obtained on crystallization from ethanol/water [M.P. 104-106°]. This product may also be called 4-(4-fluorophenyl)-6-(1-methylethyl)-2-phenyl-5-pyrimidinecarboxylic acid, ethyl ester.

Step 3: [2-phenyl-4-(4-fluorophenyl)-6-isopropylpyrimidin-5-yl]-methanol (a compound D):

To a cooled (about -10°) solution of 4.0 g of the product of step 2 above in 50 ml of dry THF is added dropwise 55 ml of a solution of 1M diisobutyl aluminium hydride in THF. The reaction mixture, after stirring at room temperature for 3 hours, is quenched at 0° with dilute hydrochloric acid and extracted several times with ether. The combined ether extracts, after washing with water, saturated brine, and drying, are filtered and evaporated in vacuo to dryness. The residue on crystallization with ethanol/water gives the product (M.P. 191-193°). This product may also be called 4-(4-fluorophenyl)-6-(1-methylethyl)-2-phenyl-5-pyrimidinemethanol.

Step 4: [2-phenyl-4-(4-fluorophenyl)-6-isopropylpyrimidin-5-yl]-formamide (a compound E):

To a cooled solution of 0.79 ml of oxalylchloride in 40 ml of $CH_2Cl_2$ at -78° is added 1.37 ml of dimethylsulfoxide in 5 ml of $CH_2Cl_2$. After stirring the reaction mixture for 10 minutes, a solution of 2.7 g of the product of step c above in 80 ml $CH_2Cl_2$ is added and the reaction mixture stirred for 40 minutes. Thereafter, 5.84 ml of triethylamine are added and the reaction mixture then gradually allowed to come to room temperature. The reaction mixture is poured into water and extracted several times with $CH_2Cl_2$. The combined $CH_2Cl_2$ extracts, after washing with water, brine, and drying, are filtered and evaporated in vacuo to dryness. The solids thus obtained are treated with distilled water and filtered and dried to give the product of this step. Recrystallization from hexane gives purified product (M.P. 137-139°). This product may also be called 4-(4-fluorophenyl)-6-(1-methylethyl)-2-phenyl-5-pyrimidinecarboxaldehyde.

Step 5: [E]-ethyl[2-phenyl-4-(4-fluorophenyl)-6-isopropylpyrimidin-5-yl]-2-propenoate (a compound F).

A solution of 2.30 g of the product of step 4 above and 2.50 g carbethoxymethylene-triphenylphosphorane in 50 ml of dry toluene is refluxed for 2 hours and then stirred at room temperature for 48 hours. Solvent is removed by evaporation in vacuo and the residue filtered over silica gel to remove $(C_6H_5)_3PO$. The filtrate on evaporation in vacuo gives a residue which on trituration with hexane gives the product of this step (M.P. 80-83°). The product may also be called [E]-3-[4-(4-fluorophenyl)-6-(1-methylethyl)-2-phenylpyrimidin-5-yl]-2-propenoic acid ethyl ester.

Step 6: [E]-[2-phenyl-4-(4-fluorophenyl-6-isopropylpyrimidin-5-yl]-2-propenol (a compound G):

To a solution of 2.2 g of the product of step 5 above in 100 ml of dry THF is added at 0° dropwise 28.2 ml 1 M DIBAL solution in THF. The reaction mixture, after stirring at room temperature for 5 hours, is quenched with dilute hydrochloric acid at 0° and extracted several times with ether. The combined ether extracts, after washing with water, saturated brine, drying, filtering and evaporating in vacuo to dryness, yields crude product as a residue. The residue, on crystallization with hexane and methyl-t-butylether, gives the product (M.P. 120-123°). The product may also be called [E]-3-[4-(4-fluorophenyl)-6-(1-methylethy)-2-phenylpyrimidin-5-yl]-2-propen-1-ol.

Step 7: [E]-[2-phenyl-4-(4-fluorophenyl)-6-isopropylpyrimidin-5-yl]-2-propenal (a compound H):

A mixture of 1.8 g of the product of step 6 above and 10.8 g activated magnesium oxide in 75 ml of dry toluene is stirred at room temperature for 18 hours. The reaction mixture is then filtered off through celite, and solvent removed in vacuo to dryness. The residue is triturated with hexane to give 1.22 g of the product [M.P. 134-137°], which may also be called [E]-3-[4-(4-fluorophenyl)-6-(1-methylethyl)-2-phenylpyrimidin-5-yl]-2-propenal.

Step 8: [E]-methyl-7-[2-phenyl-4-(4-fluorophenyl)-6-isopropylpyrimidin-5-yl]-3-keto-5-hydroxy-6-heptenoate (a compound J):

To a suspension of 0.20 g 60% sodium hydride in hexane in 20 ml of dry THF at 0° is added 0.53 ml methyl acetoacetate. After stirring for 30 minutes is added 2.98 ml 1.68 M n-butyl lithium solution in hexane, and the reaction mixture is stirred at 0° for further 20 minutes. The reaction mixture is then cooled to -78°, and to it is added dropwise a solution of 1.0 g of the product of step g above in 20 ml of dry THF. After stirring the reaction mixture at -78° for 45 minutes, dilute hydroohloric acid is added dropwise and the reaction mixture extracted several times with ether. The combined ether extracts, after washing with water and brine, are dried, filtered and evaporated i.v. to dryness. From the residue is obtained upon crystallization with hexane/ether the product (M.P. 73-76°), which may also be called [E]-7-[4-(4-fluorophenyl)-6-(1-methylethyl)-2-phenylpyrimidin-5-yl]-5-hydroxy-3-oxo-6-heptenoic acid, methyl ester.

**Example 4: [R*S*][E]-trans-[6-[2-[4-(4-fluorophenyl)-6-(1-methylethyl)-2-phenylpyrimidin-5-yl]ethenyl]-3,4,5,6-tetrahydro-4-hydroxy-2H-pyran-2-one**

($R^1$ = isopropyl; $R^2$ = phenyl; Q = 4-fluorophenyl; X = (E)-CH=CH-; Y = a group Y″ wherein $R^6$ = H and the configuration is trans [racemate])
[interconversion by lactonisation or recovery in δ-lactone form]

A solution of 0.30 g of the free acid product of Example 3 b) above and 0.29 g 1-cylohexyl-3-(2-morpholinoethyl)carbodiimide metho p-toluene-sulfonate in 5 ml of dry methylene chloride is stirred at room temperature for 2 hours. The reaction mixture is diluted with 20 ml of methylene chloride, extracted several times with water, washed with saturated brine, dried, filtered and evaporated in vacuo to dryness. The residue is chromatographed over silica gel using MTBE/hexane/acetone 7:2:1 as the solvent. The desired fractions are collected and evaporated in vacuo to dryness to give the **title product** [M.P. 185-193°].

**Example 5: [R*S*]-[E]-7-[4-(4-fluorophenyl)-6-(1-methylethyl)-2-phenylpyrimidin-5-yl]-3,5-dihydroxy-6-heptenoic acid, erythro sodium salt**

($R^1$ = isopropyl; $R^2$ = phenyl; Q = 4-fluorophenyl; X = (E)-CH=CH-; Y = a group Y′ wherein $R^6$ = H, $R^7$ = Na and the configuration is erythro [racemate])
[interconversion by saponification of δ-lactone or recovery in salt form from δ-lactone]

A solution of 0.065 g of the product of Example 4 above and 0.14 ml of 1N sodium hydroxide in 6 ml of ethanol, is stirred at room temperature for 30 minutes. Thereafter, the solvent is removed in vacuo. The residue is dissolved in 3 ml distilled water and extracted with methyl t-butyl ether. The aqueous layer is separated and lyophilized to give 60 mg of the title product [M.P. 146-155°] (> 98 % pure erythro).

**Example 6:**

Following the procedures of Example 3 a), but using in place of the 2-phenyl-4-p-fluorophenyl-5-carbethoxypyrimidine used therein an approximately equivalent amount of:

a) 2-phenyl-4-(3,4-dimethylphenyl)-5-carbethoxypyrimidine or

b) 2-phenyl-4-(3,5-dimethylphenyl)-5-carbethoxypyrimidine, there is accordingly obtained the following compounds of formula I:

**a) [R*S*]-[E]-7-[4-(3,4-dimethylphenyl)-6-(1-methylethyl)-2-phenylpyrimidin-5-yl]-3,5-dihydroxy-6-heptenoic acid, erythro methyl ester**

($R^1$ = isopropyl; $R^2$ = phenyl; Q = 3,4-dimethylphenyl; X = (E)-CH=CH-; Y = a group Y′ wherein $R^6$ = H, $R^7$ = methyl and the configuration is predominantly erythro [racemate]);
(oil) [NMR (200 MHz, $CDCl_3$):
1.3-1.38 (6H, d); 1.47-1.70 (2H, m);
2.29 (6H, s); 2.41-2.50 (m, 2H); 3.21 (1H, d);
3.43 (1H, d); 3.72 (3H, s); 4.18 (1H, m);
4.46 (1H, m); 5.48-5.60 (1H, d);
6.62-6.70 (1H, d); 7.1-7.5 (m, 2H);
8.48-8.60 (m, 2H)]
which upon treatment according to the procedures of Examples 3b), 4 and 5 yields the analogous compounds of formula $Ia^3$ (**erythro free acid**), Ib (**trans-δ-lactone**; M.P. 103-107°) and $Ia^2$ (**erythro sodium salt**; M.P. 138-151 [dec.]);
and

**b) [R*S*]-[E]-7-[4-(3,5-dimethylphenyl)-6-(1-methylethyl)-2-phenylpyrimidin-5-yl]-3,5-dihydroxy-6-heptenoic acid, erythro ethyl ester;**

($R^1$ = isopropyl; $R^2$ = phenyl; Q =3,5-dimethylphenyl; X = (E)-CH=CH-; Y = a group Y′ wherein $R^6$ = H, $R^7$ = ethyl and the configuration is predominantly erythro [racemate])
(oil) [NMR (200 MHz, $CDCl_3$):
1.18-1.69 (8H, m); 2.36 (6H, s); 2.40-2.48 (m, 2H);
3.34 (1H, d); 3.72 (1H, d); 4.46 (m, 1H);
5.46-5.57 (1H, dd); 6.61-6.70 (1H, dd);
7.0 (1H, s); 7.19-7.27 (2H, m); 7.37-7.50 (m, 3H);
8.48-8.62 (2H, m)]
which upon treatment according to the procedures of Examples 3 b), 4 and 5, accordingly yields the analogous compounds of formula $Ia^3$ (**erythro free acid**), Ib (**trans-δ-lactone**; M.P. 154-157°) and $Ia^2$ (**erythro sodium salt**; M.P. 139-150° [dec.]).

## Example 7

Carrying out the procedure of step 8 of Example 3, but using in place of the methyl acetoacetate used therein an approximately equivalent amount of ethyl acetoacetate, there is accordingly obtained the equivalent ethyl ester ketone analog, which upon reduction as described in Example 3 a) yields the **ethyl ester analog** of the methyl ester final product of Example 3 a).

Treatment of said ethyl ester final product by the procedures of Examples 3 b), 4 and 5 accordingly yields the same products as those Examples, respectively, namely the **erythro free acid**, the **trans-δ-lactone** and the **erythro sodium salt.**

## Example 8: [R*S*]-[E]-7-[4-(4-fluorophenyl)-6-(1-methylethyl)-2-phenylpyrimidin-5-yl]-3,5-dihydroxy-6-heptenoic acid, sodium salt

($R^1$ = isopropyl; $R^2$ = phenyl; Q = 4-fluorophenyl; X = (E)-CH=CH-; Y = a group Y′ wherein $R^6$ = H, $R^7$ = Na and the configuration is erythro and threo [mixture of both racemates])
[interconversion to or recovery in salt form]

Treating the methyl ester product of Example 3 a) above by the procedure of Example 5 above (hydrolysis), there is accordingly obtained the **titie product** (M.P. 117-121° [dec.]). The product consists of 93% erythro and7% threo forms.

## Example 9: [R*S*]-[E]-7-[2,6-diisopropyl-4-(4-pyridyl)pyrimidin-5-yl]-3,5-dihydroxy-6-heptenoic acid, erythro ethyl ester

($R^1$, $R^2$ = isopropyl; Q = 4-pyridyl; X = [E]-CH=CH-; Y = a group Y′ wherein $R^6$ = H, $R^7$ = ethyl and

the configuration is predominantly erythro [racemate].
[process variant a) (reduction)]

The **title compound** (M.P. 117-119° ) is obtained in a manner analogous to Example 3 a), starting from (E)-ethyl-7-[2,6-diisopropyl-4-(4-pyridyl)pyrimidin-5-yl]-3-keto-5-hydroxy-6-heptenoate (see below).

The starting material is obtained as follows:

Step 1: 2,6-diisopropyl-4-(4-pyridyl)-5-carbethoxy-1,5-dihydropyrimidine (a compound Bh):

To a solution of 24.2 g of isopropyl amidine hydrochloride in ethanol is added 16.5 g of sodium acetate and the mixture is stirred at room temperature for one half hour. To the mixture is then added 48 g of a solution of 5-methyl-3-oxo-2-(4-pyridylmethylene)pentanoic acid, ethyl ester in ethanol, and the resulting mixture refluxed for about 24 hours. The mixture is then poured into a liter of water, extracted 3 times with ether, the combined extracts are dried, filtered and evaporated to obtain crude product as an orange-red oily residue. The residue is refined by flash chromatographing, eluting with methanol/MTBE (1:9 v/v) to obtain refined product (yellow solid; M.P. 103.5-105° ).

Step 2: 2,6-diisopropyl-4-(4-pyridyl)pyrimidin-5-yl]formamide (a compound E):

Treating the product of step 1 by the methods of steps 2, 3 and 4 of Example 3 (to in turn aromatize, reduce and oxidize the product) yields the aldehyde product.

Step 3: [E]-3-[2,6-diisopropyl-4-(4-pyridyl)pyrimidin-5-yl]-2-propenal (a compound H):

Under argon, 0.95 g (0.67 ml) of cis-1-bromo-2-ethoxyethylene is added to 30 ml of dry THF and the mixture cooled to -78° . To the cooled mixture is added by syringe 6.7 ml of 1.7 M t-butyl lithium and the mixture stirred for one hour.

To the mixture, at -78° , is then added, dropwise, 0.79 g of the aldehyde product of step 2 above, and the resulting mixture stirred for 20 minutes at -78° . The mixture is then quenched with 2 ml of saturated aqueous ammonium chloride (at -78° ). Two ml of water is then added, and the mixture extracted twice with ether. The combined ether extracts are dried, filtered and evaporated to obtain a residue.

The residue is dissolved in water/THF (1:9), 1.6 g of p-toluene sulfonic acid added, and the resulting mixture stirred for 20 minutes. The mixture is then poured into water, the pH adjusted to 8 (with dilute sodium hydroxide) and extracted twice with ether. The combined ether extracts are dried, filtered and evaporated to obtain as a residue crude product (as a white solid which is slightly soluble in hexane at room temperature; M.P. 113.5-114.5° ).

Step 4:

Treating the aldehyde of step 3 above as in step 8 of Example 3 above, but using ethyl acetoacetate instead of methyl acetoacetate, there is accordingly obtained the 3-keto-5-hydroxy product used as a starting material of this Example.

**Example 10:**

The following further compounds of formula I are obtained in a manner analogous to Example 3 a) [process variant a) (reduction)]:

| Sub-example No. | $R^1$ | $R^2$ | Q | X | Y | M.P. |
|---|---|---|---|---|---|---|
| 10a[1)] | isopropyl | t-butyl | 4-pyridyl | (E)-CH=CH- | Y' wherein $R^6$ = H $R^7$ = ethyl and the configuration is predominantly erythro (racemate) | |
| 10b[1)] | dito | methyl | dito | dito | dito | |
| 10c[1)] | dito | $-N(CH_3)_2$ | dito | dito | dito | |
| 10d[2)] | dito | t-butyl | 4-fluoro-phenyl | dito | dito | 85-87° |
| 10e[2)] | dito | methyl | dito | dito | dito | foam (NMR[4)]) |
| 10f[2)] | dito | isopropyl | dito | dito | dito | foam (NMR[5)]) |
| 10g[2)] | dito | $-N(CH_3)_2$ | dito | dito | dito | resinous oil (NMR[6)]) |
| 10g[3)] | methyl | methyl | dito | dito | dito | oil (NMR[7)]) |

1) The corresponding starting material is obtained in a manner analogous to Example 9, steps 1 to 4, whereby for step 1 the corresponding amidine hydrochloride salt wherein $R^2$ is t-butyl, methyl or, respectively, dimethylamino is reacted with 5-methyl-3-oxo-2-(4-pyridylmethylene)pentanoic acid, ethyl ester

2) The corresponding starting material is obtained in a manner analogous to Example 9, steps 1 to 4, whereby for step 1 the corresponding amidine hydrochloride salt wherein $R^2$ is t-butyl, methyl, isopropyl or, respectively, dimethylamino is reacted with 5-methyl-3-oxo-2-[(4-fluorophenyl)methylene]pentanoic acid, ethyl ester

3) Using for step 1 2-(4-fluorophenyl)methylene-3-oxo-butyric acid ethyl ester instead of 5-methyl-3-oxo-2-(4-pyridylmethylene)pentanoic acid ethyl ester and methyl amidine hydrochloride instead of isopropyl amidine hydrochloride

4) (200 MHz, $CDCl_3$) 1.23-1.26 (6H, d); 1.38-1.64 (m, 2H); 2.35-2.48 (m, 2H); 2.71 (3H, d); 3.47 (1H, m); 3.68 (1H, m); 4.12-4.23 (1H, m); 4.42 (1H, m); 5.37-5.48 (1H, dd); 6.56-6.64 (1H, dd); 7.02-7.13 (2H, mm); 7.44-7.57 (2H, m)

5) (200 MHz, $CDCl_3$) 1.25-1.28 (6H, d); 1.35-1.38 (6H, d); 2.38-2.50 (2H, m); 3.12-3.28 (1H, m); 3.28-3.41 (1H, m); 3.42 (1H, d); 3.69 (1H, d); 4.44 (1H, m); 5.39-5.51 (1H, dd); 6.58-6.67 (1H, dd); 7.03-7.12 (2H, m); 7.51-7.62 (2H, m)

6) (200 MHz, $CDCl_3$) 1.18-1.32 (1H, m); 1.41-1.67 (2H, m); 2.40-2.48 (2H, m); 3.13 (1H, d); 3.22 (6H, s); 3.68 (1H, d); 4.13-4.23 (2H, m); 4.40 (1H, m); 5.23-5.26 (1H, dd); 6.48-6.57 (1H, dd); 7.00-7.08 (2H, m); 7.52-7.59 (2H, m)

7) (200 MHz, $CDCl_3/CD_3OD$) 1.22-1.37 (3H, m); 1.46-1.70 (2H, m); 2.40-2.48 (2H, m); 2.56 (3H, s); 2.69 (3H, s); 5.54-5.66 (1H, dd); 6.43-6.53 (1H, d); 7.04-7.21 (2H, m); 7.38-7.65 (2H, m)

**Example 11:**

The following sodium salts are obtained in a manner analogous to Example 5 (hydrolysis), starting from the corresponding ester of Example 9 or 10:

| Sub-example No. | $R^1$ | $R^2$ | Q | X | Y | Starting from ester of Example No. | M.P. |
|---|---|---|---|---|---|---|---|
| 11a | isopropyl | isopropyl | 4-pyridyl | (E)-CH=CH- | Y' wherein $R^6$=H $R^7$=Na and the configuration is predominantly (>98 %) erythro (racemate) | 9 | 224-225° (dec.) |
| 11b | dito | t-butyl | dito | dito | dito | 10a | |
| 11c | dito | methyl | dito | dito | dito | 10b | |
| 11d | dito | $-N(CH_3)_2$ | dito | dito | dito | 10c | |
| 11e | dito | t-butyl | 4-fluoro-phenyl | dito | dito | 10d | foam (NMR[1)]) |
| 11f | dito | methyl | dito | dito | dito | 10e | foam (NMR[2)]) |
| 11g | dito | isopropyl | dito | dito | dito | 10f | 172-178° (dec.) |
| 11h | dito | $-N(CH_3)_2$ | dito | dito | dito | 10g | 210-212° (dec.) |
| 11i | methyl | methyl | dito | dito | dito | 10h | foam (NMR[3)]) |

1) (200 MHz, $CD_3OD$) 1.21-1.25 (6H, d); 1.37 (9H, s); 1.42-1.73 (m, 2H); 3.38-3.42 (m, 1H); 4.26-4.37 (m, 1H); 5.47-5.67 (1H, dd); 6.55-6.63 (1H, dd); 7.03-7.20 (2H, m); 7.52-7.69 (2H, m)

2) (200 MHz, $CD_3OD$) 1.22-1.27 (6H, d); 1.36-1.71 (2H, m); 2.12-2.44 (2H, m); 2.66 (3H, s); 3.40-3.53 (1H, m); 3.86 (1H, m); 4.30 (1H, m); 5.46-5.57 (1H, dd); 6.52-6.59 (1H, dd); 7.12-7.22 (2H, m); 7.51-7.58 (2H, m)

3) (200 MHz, $CD_3OD$) 1.43-1.77 (2H, m); 2.57 (3H, s); 2.65 (3H, s); 5.62-5.74 (1H, dd); 6.43-6.53 (1H, d); 7.13-7.26 (2H, m); 7.55-7.67 (2H, m)

**Example 12**

A representative formulation of a compound of this invention for administration orally 3 times per day is prepared by conventional techniques for encapsulation in a hard gelatin capsule and is:

| | |
|---|---|
| Compound of Example 1b | 10 mg |
| Corn starch | 238 mg |
| Magnesium stearate | 2 mg |

## Claims

1. A compound of formula I

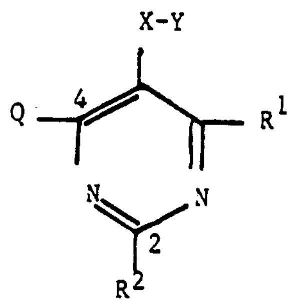

I

wherein
either
$R^1$ and $R^2$ independently are:
$C_{1-6}$alkyl not containing an asymmetric carbon atom;
$C_{3-6}$cycloalkyl; or

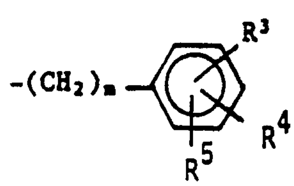

wherein
m is 0, 1, 2 or 3;
$R^3$ is hydrogen, $C_{1-3}$alkyl, n-butyl, i-butyl, t-butyl, $C_{1-3}$alkoxy, n-butoxy, i-butoxy, trifluoromethyl, fluoro, chloro, phenoxy or benzyloxy;
$R^4$ is hydrogen, $C_{1-3}$alkyl, $C_{1-3}$alkoxy, trifluoromethyl, fluoro, chloro, phenoxy or benzyloxy; and
$R^5$ is hydrogen, $C_{1-2}$alkyl, $C_{1-2}$alkoxy, fluoro or chloro;
with the provisos that
- not more than one of $R^3$ and $R^4$ is trifluoromethyl;
- not more than one of $R^3$ and $R^4$ is phenoxy; and
- not more than one of $R^3$ and $R^4$ is a benzyloxy;
or
$R^1$ is as defined above and

$R^2$ is: benzyloxy;

benzylthio;

$-N(R^8)_2$ wherein either each $R^8$ independently is $C_{1-4}$alkyl not containing an asymmetric carbon atom or both $R^8$ together with the nitrogen atom form part of a 5-,6- or 7-membered optionally substituted ring optionally containing one or more further heteroatoms (ring B); or

Q wherein Q is as defined below;

Q is Q′ or Q″ wherein

Q′ is a heterocyclic group optionally mono- or independently disubstituted by $C_{1-2}$alkyl or $C_{1-2}$alkoxy and

Q″ is either Q″$_a$ wherein Q″$_a$ is

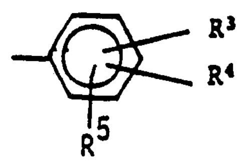

wherein

$R^3$, $R^4$ and $R^5$ are as defined above, including the provisos thereto,

or Q″$_b$ wherein Q″$_b$ is

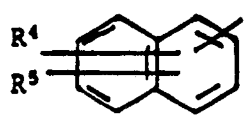

wherein

$R^4$ and $R^5$ are as defined above;

X is either ethylene or vinylene; and

Y is: - a group Y′ of formula

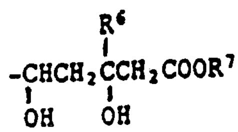

wherein

$R^6$ is hydrogen or $C_{1-3}$alkyl; and

$R^7$ is hydrogen, an ester group ($R^{7'}$) or a cation (M);

- a group Y″ of formula

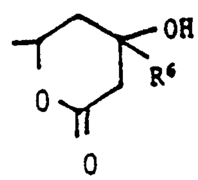

wherein $R^6$ is as defined above; or

- a group Y‴ of formula

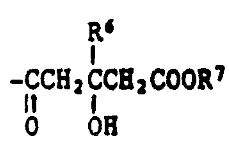

wherein
$R^6$ and $R^7$ are as defined above;
with the proviso that when Y is a group $Y'''$,
then X is vinylene and/or
$R^6$ is $C_{1-3}$ alkyl;
in free acid form, or in the form of an ester of δ-lactone thereof, or in salt form as appropriate.

2. A compound according to claim 1 wherein the hydroxy groups in the 3- and 5-positions of the group $Y'$ have the erythro configuration, in racemic form; in free acid form, or in the form of an ester or δ-lactone thereof, or in salt form as appropriate.

3. A compound according to claim 2 in 3R,5S enantiomer form; in free acid form, or in the form of an ester or δ-lactone theeof, or in salt form as appropriate.

4. A compound according to claim 1 of formula Is

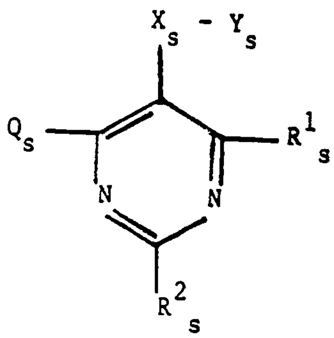

Is

wherein
$R^1_s$ is $(C_{1-3})$alkyl,
$R^2_s$ is $(C_{1-3})$alkyl; phenyl; 4-morpholinyl; or di$(C_{1-2})$alkylamino;
$Q_s$ is - phenyl optionally monosubstituted in the 4-position by fluorine or disubstituted in the 3-position and in the 4- or 5-position by $C_{1-2}$alkyl; or
- 4-pyridyl;
$X_s$ is (E)-vinylene; and
$Y_s$ is - a group $Y'_s$ of formula-$CH(OH)CH_2CH(OH)CH_2COOR^7_s$
wherein $R^7_s$ is hydrogen, $(C_{1-4})$alkyl or
an alkali cation; or
- a group $Y''_s$ of formula

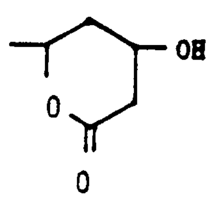

in free acid form, or in the form of an ester or δ-lactone thereof, or in salt form as appropriate.

5. A compound according to claim 1 selected from:
-(3R,5S)-[E]-7-[4-(4-fluorophenyl)-6-(1-methylethyl)-2-(dimethylamino)pyrimidin-5-yl]-3,5-dihydroxy-6-heptenoic acid, (1,1-dimethylethyl) ester; or sodium salt;
-(3R,5S)-[E]-7-[4-(4-fluorophenyl)-6-(1-methylethyl)-2-(4-morpholinyl)pyrimidin-5-yl]-3,5-dihydroxy-6-heptenoic acid, tert-butyl ester; or sodium salt;
-[R*S*]-[E]-7-[4-(4-fluorophenyl)-6-(1-methylethyl)-2-phenyl-pyrimidin-5-yl]-3,5-dihydroxy-6-heptenoic acid, erythro methyl ester; erythro sodium salt; or erythro free acid;
- [R*S*]-[E]-trans-[6-[2-[4-(4-fluorophenyl)-6-(1-methylethyl)-2-phenylpyrimidin-5-yl]ethenyl]-3,4,5,6-tetrahydro-4-hydroxy-2H-pyran-2-one;
- [R*S*]-[E]-7-[4-(4-fluorophenyl)-6-(1-methylethyl)-2-phenylpyrimidin-5-yl]-3,5-dihydroxy-6-heptenoic acid, erythro sodium salt;

- [R*S*]-[E]-7-[4-(3,4-dimethylphenyl)-6-(1-methylethyl)-2-phenylpyrimidin-5-yl]-3,5-dihydroxy-6-heptenoic acid, erythro methyl ester; erythro free acid; trans δ-lactone; or erythro sodium salt;
- [R*S*]-[E]-7-[4-(3,5-dimethylphenyl)-6-(1-methylethyl)-2-phenylpyrimidin-5-yl]-3,5-dihydroxy-6-heptenoic acid, erythro methyl ester; erythro free acid; trans δ-lactone; or erythro sodium salt;
- [R*S*]-[E]-7-[4-(4-fluorophenyl)-6-(1-methylethyl)-2-phenylpyrimidin-5-yl]-3,5-dihydroxy-6-heptenoic acid, erythro ethyl ester; erythro free acid; trans δ-lactone; or erythro sodium salt;
- [R*S*]-[E]-7-[4-(4-fluorophenyl)-6-(1-methylethyl)-2-phenylpyrimidin-5-yl]-3,5-dihydroxy-6-heptenoic acid, sodium salt;

-[R*S*]-[E]-7-[2,6,diisopropyl-4-(4-pyridyl)pyrimidin-5-yl]-3,5-dihydroxy-6-heptenoic acid, erythro ethyl ester;
- the compounds of formula I wherein

X is (E)-CH=CH-,
Y is Y′ wherein $R^6$ is H, $R^7$ is ethyl and the configuration is predominantly erythro (racemate) and either
$R^1$ is isopropyl,
$R^2$ is t-butyl, methyl or $-N(CH_3)_2$ and
Q is either 4-pyridyl or 4-fluorophenyl,
or
$R^1$ and $R^2$ are either both isopropyl or both methyl and
Q is 4-fluorophenyl; and

- the compounds of formula I wherein

X is (E)-CH=CH-,
Y is Y′ wherein $R^6$ is hydrogen, $R^7$ is Na and the configuration is predominantly erythro (racemate) and either
$R^1$ is isopropyl,
$R^2$ is isopropyl, tert-butyl, methyl or $-N(CH_3)_2$ and
Q is either 4-pyridyl or 4-fluorophenyl,
or
$R^1$ and $R^2$ are methyl and
Q is 4-fluorophenyl.

6. A pharmaceutical composition comprising a compound according to claim 1 in free acid form or in the form of a physiologically hydrolysable and -acceptable ester or a δ-lactone thereof or in pharmaceutically acceptable salt form as appropriate, together with a pharmaceutically acceptable diluent or carrier.

7. A compound according to claim 1 in free acid form or in the form of a physiologically-hydrolysable and -acceptable ester or a δ-lactone thereof or in pharmaceutically acceptable salt form as appropriate, for use as a pharmaceutical.

8. A compound according to claim 1 in free acid form or in the form of a physiologically-hydrolysable and -acceptable ester or a δ-lactone thereof or in pharmaceutically acceptable salt form as appropriate, for use in inhibiting cholesterol biosynthesis or treating atherosclerosis.

9. A process for preparing a compound according to claim 1 which comprises hydrolysing a compound of formula I in ester or δ-lactone form or esterifying or lactonising a compound of formula I in free acid form and when a free carboxyl group is present recovering the compound obtained in free acid form or in the form of a salt as appropriate.

10. A process for preparing a compound according to claim 1 which comprises

a) when $R^6$ is hydrogen,

reducing a corresponding compound of formula AAA

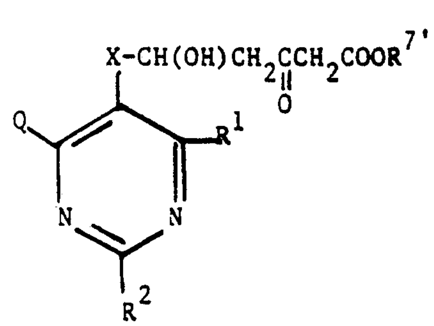

AAA

wherein $R^1$, $R^2$, Q, X and $R^{7'}$ are as defined in claim 1, or

b) when $R^6$ is $(C_{1-3})$alkyl,

hydrolyzing a corresponding compound of formula BBB

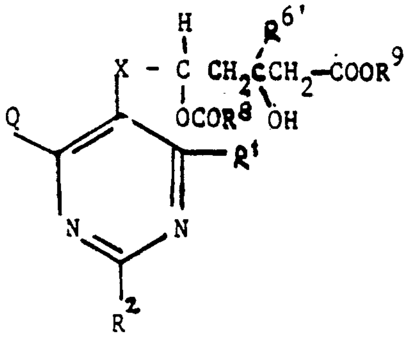

BBB

wherein $R^1$, $R^2$, Q and X are as defined in claim 1, $R^{6'}$ is $C_{1-3}$alkyl and $R^8$ and $R^9$ are part of an ester-forming group, or

c) when $R^6$ is hydrogen and X is (E)-CH=CH-,

deprotecting a corresponding compound of formula CCC

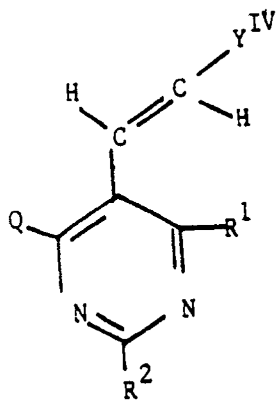

CCC

wherein $R^1$, $R^2$ and Q are as defined in claim 1 and $Y^{IV}$ is

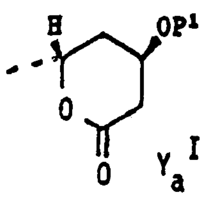

$Y_a^{IV}$

or

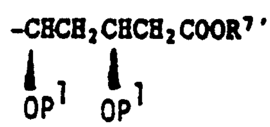

$Y_b^{IV}$

wherein $R^{7'}$ is as defined in claim 1 and

$P^1$ is a protecting group, or

d) when Y is a group of formula $Y'''$ as defined in claim 1, oxidizing a corresponding compound of formula DDD

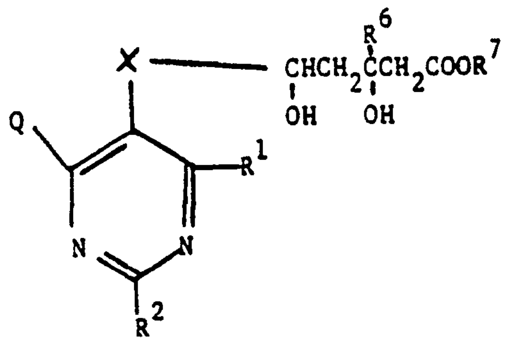

DDD

wherein $R^1$, $R^2$, $R^6$, $R^7$, X and Q are as defined in claim 1; and recovering the resultant compounds of formula I in free acid form, or in the form of an ester or δ-lactone thereof, or in salt form as appropriate.

CLAIMS FOR THE FOLLOWING CONTRACTING STATES ES AND GR:

1. A process for preparing a compound of formula I

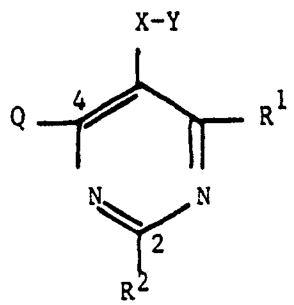

I

wherein
either
$R^1$ and $R^2$ independently are:
$C_{1-6}$alkyl not containing an asymmetric carbon atom;
$C_{3-6}$cycloalkyl; or

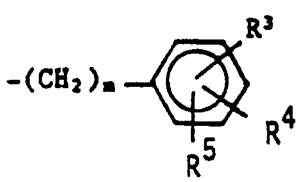

wherein
m is 0, 1, 2 or 3;
$R^3$ is hydrogen, $C_{1-3}$alkyl, n-butyl, i-butyl, t-butyl, $C_{1-3}$alkoxy, n-butoxy, i-butoxy, trifluoromethyl, fluoro, chloro, phenoxy or benzyloxy;
$R^4$ is hydrogen, $C_{1-3}$alkyl, $C_{1-3}$alkoxy, trifluoromethyl, fluoro, chloro, phenoxy or benzyloxy; and
$R^5$ is hydrogen, $C_{1-2}$alkyl, $C_{1-2}$alkoxy, fluoro or chloro;
with the provisos that
- not more than one of $R^3$ and $R^4$ is trifluoromethyl;
- not more than one of $R^3$ and $R^4$ is phenoxy; and
- not more than one of $R^3$ and $R^4$ is a benzyloxy;
or
$R^1$ is as defined above and
$R^2$ is: benzyloxy;

benzylthio;

$-N(R^8)_2$ wherein either each $R^8$ independently is $C_{1-4}$alkyl not containing an asymmetric carbon atom or both $R^8$ together with the nitrogen atom form part of a 5-,6- or 7-membered optionally substituted ring optionally containing one or more further heteroatoms (ring B); or

Q wherein Q is as defined below;

Q is Q′ or Q″ wherein

Q′ is a heterocyclic group optionally mono- or independently disubstituted by $C_{1-2}$alkyl or $C_{1-2}$alkoxy and

Q″ is either Q″a wherein Q″a is

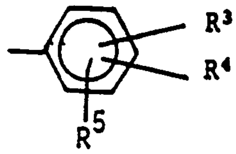

wherein

$R^3$, $R^4$ and $R^5$ are as defined above, including the provisos thereto,

or Q″b wherein Q″b is

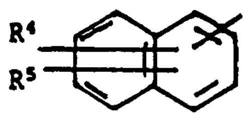

wherein

$R^4$ and $R^5$ are as defined above;

X is either ethylene or vinylene; and

Y is: - a group Y′ of formula

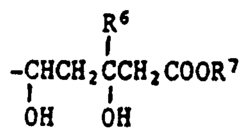

wherein

$R^6$ is hydrogen or $C_{1-3}$alkyl; and

$R^7$ is hydrogen, an ester group ($R^{7'}$) or a cation (M);

- a group Y″ of formula

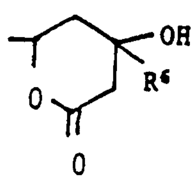

wherein $R^6$ is as defined above; or

- a group Y‴ of formula

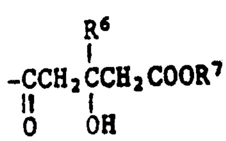

wherein

$R^6$ and $R^7$ are as defined above;

with the proviso that when Y is a group Y‴,
then X is vinylene and/or
$R^6$ is $C_{1-3}$ alkyl;
in free acid form, or in the form of an ester or δ-lactone thereof, or in salt form as appropriate, which comprises

a) when $R^6$ is hydrogen,
reducing a corresponding compound of formula AAA

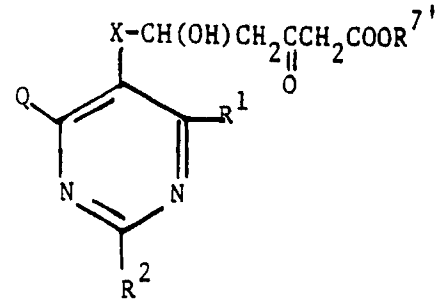

AAA

wherein $R^1$, $R^2$, Q, X and $R^{7'}$ are as defined in this claim, or

b) when $R^6$ is $(C_{1-3})$alkyl,
hydrolyzing a corresponding compound of formula BBB

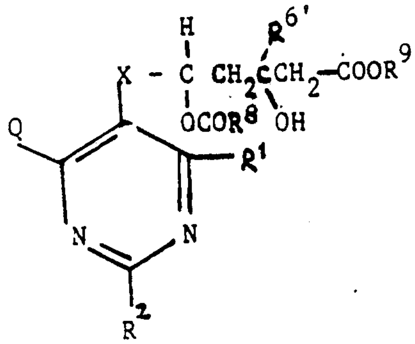

BBB

wherein $R^1$, $R^2$, Q and X are as defined in this claim, $R^{6'}$ is $C_{1-3}$alkyl and $R^8$ and $R^9$ are part of an ester-forming group, or

c) when $R^6$ is hydrogen and X is (E)-CH=CH-,
deprotecting a corresponding compound of formula CCC

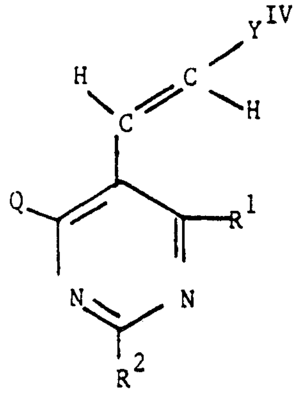

CCC

wherein $R^1$, $R^2$ and Q are as defined in this claim and $Y^{IV}$ is

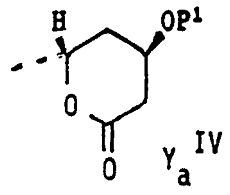

or

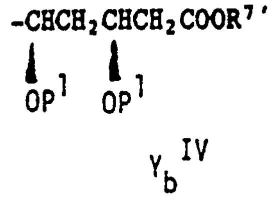

wherein $R^{7'}$ is as defined in this claim and $P^1$ is a protecting group, or

d) when Y is a group of formula Y‴ as defined in this claim, oxidizing a corresponding compound of formula DDD

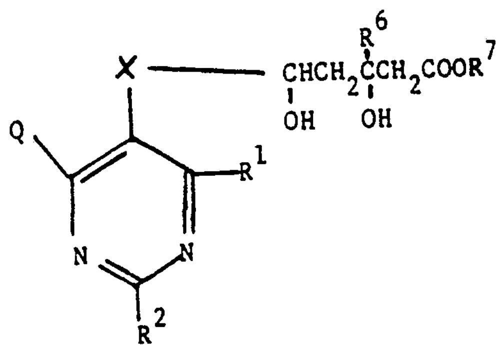

DDD

wherein $R^1$, $R^2$, $R^6$, $R^7$, X and Q are as defined in this claim, and recovering the resultant compounds of formula I in free acid form, or in the form of an ester or δ-lactone thereof, or in salt form as appropiate.

2. A process for preparing a compound of formula I as defined in claim 1 which comprises hydrolysing a compound of formula I in ester or δ-lactone form or esterifying or lactonising a compound of formula I in free acid form and when a free carboxyl group is present recovering the compound obtained in free acid form or in the form of a salt as appropriate.

3. A process according to claim 1 or 2 preparing a compound of formula I wherein the hydroxy groups in the 3- and 5-positions of the group Y′ have the erythro configuration, in racemic form; in free acid form, or in the form of an ester or δ-lactone thereof, or in salt form as appropriate.

4. A process according to claim 1 or 2 for preparing a compound of formula I in 3R,5S enantiomer form; in free acid form, or in the form of an ester or δ-lactone thereof, or in salt form as appropriate.

5. A process according to claim 1 or 2 for preparing a compound of formula Is

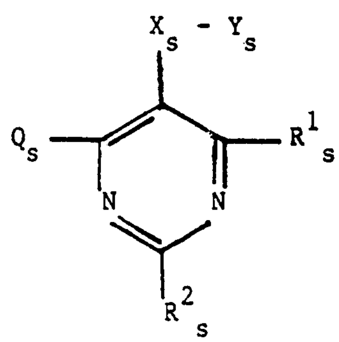

Is

wherein

$R^1_s$ is $(C_{1-3})$alkyl,

$R^2_s$ is $(C_{1-3})$alkyl; phenyl; 4-morpholinyl; or di$(C_{1-2})$alkylamino;

$Q_s$ is - phenyl optionally monosubstituted in the 4-position by fluorine or disubstituted in the 3-position and in the 4- or 5-position by $C_{1-2}$alkyl; or

- 4-pyridyl;

$X_s$ is (E)-vinylene; and

$Y_s$ is - a group $Y'_s$ of formula $-CH(OH)CH_2CH(OH)CH_2COOR^7{}_s$

wherein $R^7{}_s$ is hydrogen, $(C_{1-4})$alkyl or an alkali cation; or
- a group $Y''_s$ of formula

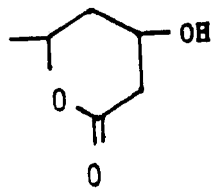

in free acid form, or in the form of an ester or δ-lactone thereof, or in salt form as appropriate.

6. A process according to claim 1 or 2 for preparing a compound selected from:
- (3R,5S)-[E]-7-[4-(4-fluorophenyl)-6-(1-methylethyl)-2-(dimethylamino)pyrimidin-5-yl]-3,5-dihydroxy-6-heptenoic acid, (1,1-dimethylethyl) ester; or sodium salt;
- (3R,5s)-[E]-7-[4-(4-fluorophenyl)-6-(1-methylethyl)-2-(4-morpholinyl)pyrimidin-5-yl]-3,5-dihydroxy-6-heptenoic acid, tert-butyl ester; or sodium salt;
- [R*S*]-[E]-7-[4-(4-fluorophenyl)-6-(1-methylethyl)-2-phenyl-pyrimidin-5-yl]-3,5-dihydroxy-6-heptenoic acid, erythro methyl ester; erythro sodium salt; or erythro free acid;
- [R*S*]-[E]-trans-[6-[2-[4-(4-fluorophenyl)-6-(1-methylethyl)-2-phenylpyrimidin-5-yl]ethenyl]-3,4,5,6-tetrahydro-4-hydroxy-2H-pyran-2-one;
- [R*S*]-[E]-7-[4-(4-fluorophenyl)-6-(1-methylethyl)-2-phenylpyrimidin-5-yl]-3,5-dihydroxy-6-heptenoic acid, erythro sodium salt;
- [R*S*]-[E]-7-[4-(3,4-dimethylphenyl)-6-(1-methylethyl)-2-phenylpyrimidin-5-yl]-3,5-dihydroxy-6-heptenoic acid, erythro methyl ester; erythro free acid; trans δ-lactone; or erythro sodium salt;
- [R*S*]-[E]-7-[4-(3,5-dimethylphenyl)-6-(1-methylethyl)-2-phenylpyrimidin-5-yl]-3,5-dihydroxy-6-heptenoic acid, erythro methyl ester; erythro free acid; trans δ-lactone; or erythro sodium salt;
- [R*S*]-[E]-7-[4-(4-fluorophenyl)-6-(1-methylethyl)-2-phenylpyrimidin-5-yl]-3,5-dihydroxy-6-heptenoic acid, erythro ethyl ester; erythro free acid; trans δ-lactone; or erythro sodium salt;
- [R*S*]-[E]-7-[4-(4-fluorphenyl)-6-(1-methylethyl)-2-phenylpyrimidin-5-yl]-3,5-dihydroxy-6-heptenoic acid, sodium salt;
- [R*S*]-[E]-7-[2,6-diisopropyl-4-(4-pyridyl)pyrimidin-5-yl]-3,5-dihydroxy-6-heptenoic acid, erythro ethyl ester;
- the compounds of formula I wherein
X is (E)-CH=CH-,
Y is Y′ wherein $R^6$ is H, $R^7$ is ethyl and the configuration is predominantly erythro (racemate) and
either
$R^1$ is isopropyl,
$R^2$ is t-butyl, methyl or $-N(CH_3)_2$ and
Q is either 4-pyridyl or 4-fluorophenyl,
or
$R^1$ and $R^2$ are either both isopropyl or both methyl and
Q is 4-fluorophenyl; and
- the compounds of formula I wherein
X is (E)-CH=CH-,
Y is Y′ wherein $R^6$ is hydrogen, $R^7$ is Na and the configuration is predominantly erythro (racemate) and
either
$R^1$ is isopropyl,
$R^2$ is isopropyl, tert-butyl, methyl or $-N(CH_3)_2$ and
Q is either 4-pyridyl or 4-fluorophenyl,
or
$R^1$ and $R^2$ are methyl and
Q is 4-fluorophenyl.

7. A process for the preparation of a pharmaceutical composition which comprises mixing a compound of formula I in free acid form or in the form of a physiologically hydrolysable and -acceptable ester or a δ-lactone thereof or in pharmaceutically acceptable salt form as appropriate, with a pharmaceutically acceptable diluent or carrier.

European Patent Office

# EUROPEAN SEARCH REPORT

Application Number

EP 89 10 3931

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | DE-A-3 823 045 (HOECHST)(19-01-1989) * Whole document * & EP-A-307 342 (15-03-1989),(Cat. E) | 1,3,4,6 | C 07 D 405/06<br>C 07 D 239/42<br>C 07 D 239/26<br>C 07 D 401/04<br>A 61 K 31/505 |
| E | EP-A-0 308 736 (NISSAN)(29-03-1989)) * Page 1, lines 14-21; claims * | 1,3,4,6 | |
| | | | **TECHNICAL FIELDS SEARCHED (Int. Cl.4)** |
| | | | C 07 D 405/00<br>C 07 D 239/00 |

The present search report has been drawn up for all claims

| Place of search | Date of completion of the search | Examiner |
|---|---|---|
| THE HAGUE | 02-02-1990 | FRANCOIS J.C.L. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document